# EUROPEAN PATENT APPLICATION

(11) **EP 4 083 624 A1**
(43) Date of publication of application: **02.11.2022**
(21) Application number: 20906586.1
(22) Date of filing: 22.12.2020
(51) Int. Cl.: G01N 33/53, G01N 33/574

(54) **METHOD FOR ASSISTING DIAGNOSIS OF METASTATIC CASTRATION-RESISTANT PROSTATE CANCER**

(30) Priority: 27.12.2019 JP 2019237968
(71) Applicant: FUJIFILM Corporation, Tokyo 106-8620 (JP); Tokyo Metropolitan Geriatric Hospital and Institute of Gerontology, Tokyo 173-0015 (JP); National University Corporation Tokai National Higher Education and Research System, Nagoya-shi, Aichi 464-8601 (JP)
(72) Inventor: KAWAKAMI Kyojiro, Tokyo 173-0015 (JP); FUJITA Yasunori, Tokyo 173-0015 (JP); ITO Masafumi, Tokyo 173-0015 (JP); MIZUTANI Kosuke, Gifu-shi Gifu 501-1193 (JP); ISHIKAWA Tomokazu, Amagasaki-shi Hyogo 661-0963 (JP)
(74) Representative: Hoffmann Eitle
(86) International application number: PCT/JP2020/047963
(87) International publication number: WO 2021/132245

(57) **Abstract**

An object of the present invention is to provide a new prostate cancer marker with which metastatic castration resistant prostate cancer can be easily and specifically diagnosed and the state of disease of the metastatic castration resistant prostate cancer is monitored.

The present invention relates to a method of assisting the diagnosis of metastatic castration resistant prostate cancer, including measuring an amount of an extracellular vesicle having phosphatidylserine and a prostate specific membrane antigen in a biological specimen derived from a subject; and determining whether or not the subject has metastatic castration resistant prostate cancer by using, as an indicator, the amount of the extracellular vesicle having phosphatidylserine and a prostate specific membrane antigen.

## Description

### BACKGROUND OF THE INVENTION

### 1. Field of the Invention

The present invention relates to a method of assisting diagnosis of metastatic castration resistant prostate cancer.

### 2. Description of the Related Art

Hormone therapy is one of treatment methods for prostate cancer. However, it is not a radical treatment method, and thus in some prostate cancer patients, the prostate cancer proceeds sooner or later to hormone therapy resistant prostate cancer (castration resistant prostate cancer, hereinafter abbreviated as "CRPC"). CRPC is generally diagnosed with an increase in the prostate specific antigen (hereinafter abbreviated as "PSA") which is a prostate cancer marker in the related art; however, recently, it has also been pointed out that the progress of the state of disease without correlation with an increase in PSA.

As a result, in the treatment process of CRPC, it is recommended to monitor the progress of the state of disease including metastasis by using not only the PSA measurement but also imaging diagnosis in combination.

A prostate specific membrane antigen (hereinafter abbreviated as "PSMA") is expressed on the cell membrane of the prostate, and it has been known that in the tissues of prostate cancer patients, the higher the grade of malignancy of the specimen, the stronger the stainability of PSMA.

By the way, it is known that in the inside of particles of extracellular vesicles, proteins and nucleic acids such microRNAs are present, and the extracellular vesicle is responsible for substance transportation between cells. Extracellular vesicles are also secreted into body fluids such as blood, and proteins, microRNAs, and the like in the extracellular vesicle is attracting attention as diagnostic markers for diseases. According to Mizutani et al., exosomes have been separated from plasma derived from a healthy subject, a prostate cancer patient without metastasis, a patient with progressive prostate cancer, and a CRPC patient by using anti-PSMA antibody-immobilized beads and subjecting the exosomes to Western blotting using an anti-CD9 antibody. As a result, they have reported that the amount of exosomes increases in the prostate cancer patient and the CRPC patient as compared with the healthy subject (Kosuke Mizutani et al., Anticancer Research, 2014, 34(7), p.3419-3423).

### SUMMARY OF THE INVENTION

However, CRPC-specific cancer markers and biomarkers for diagnosing CRPC metastasis are unknown.

In addition, although there are documents in which an attempt was made to measure the PSMA concentration in blood (Zhen Xiao et al., Cancer Res., 2001, 61(16), p:6029-6033; Vaclav Navratil et al., Nucleic Acids Res., 2017, 45(2):el0. doi: 10.1093/nar/gkw853; and the like), it is difficult to measure PSMA in the blood with the technique in the related art since the amount of PSMA in the blood is very small. As a result, there is no consensus on the relationship between the concentration of PSMA in the blood and the prostate cancer. In Kosuke Mizutani et al., Anticancer Research, 2014, 34(7), p.3419-3423, no particular test has been carried out on patients with metastatic CRPC, either. Further, the method of checking the amount of exosomes described in Kosuke Mizutani et al., Anticancer Research, 2014, 34(7), p.3419-3423 has a problem in that the procedure is complicated in a case of being used in the field of clinical examination where rapid diagnosis is required.

In consideration of the above, an object of the present invention is to develop a new prostate cancer marker with which metastatic CRPC can be easily and specifically diagnosed and the state of disease of the metastatic CRPC is monitored.

The present invention has been made for the purpose of achieving the above object and includes the following aspects.
[1] A method of assisting diagnosis of metastatic CRPC, comprising measuring an amount of an extracellular vesicle having phosphatidylserine and PSMA in a biological specimen derived from a subject; and determining whether or not the subject has metastatic CRPC by using, as an indicator, the amount of the extracellular vesicle having phosphatidylserine and PSMA.
[2] The method according to [1], in which the determination is determining that the subject has metastatic CRPC in a case where the amount of the extracellular vesicle having phosphatidylserine and PSMA is equal to or larger than a reference value.
[3] The method according to [1] or [2], in which the measurement is a measurement in which a substance having an affinity for phosphatidylserine and an antibody having an affinity for PSMA are used.
[4] The method according to any one of [1] to [3], in which the measurement includes the following steps (i) to (iii);
   (i) a step of obtaining an extracellular vesicle from a biological specimen derived from a subject,
   (ii) a step of bringing the extracellular vesicle obtained in the step (i) into contact with a substance having an affinity for phosphatidylserine and an antibody having an affinity for PSMA, to form a complex 2 of the substance having an affinity for phosphatidylserine, the extracellular vesicle having phosphatidylserine and PSMA, and the antibody having an affinity for PSMA, and
   (iii) a step of measuring an amount of the complex 2 obtained in the step (ii) to measure an amount of the extracellular vesicle having phosphatidylserine and PSMA.
[5] The method according to any one of [1] to [4], in which the measurement includes the following steps (i) to (iii);
   (i) a step of bringing a biological specimen derived from a subject into contact with a substance having an affinity for phosphatidylserine to form a complex 1 of an extracellular vesicle having phosphatidylserine in the biological specimen and the substance having an affinity for phosphatidylserine, subsequently separating the extracellular vesicle having phosphatidylserine from the complex 1, and acquiring the extracellular vesicle having phosphatidylserine,
   (ii) a step of bringing the extracellular vesicle having phosphatidylserine, obtained in the step (i), into contact with a substance having an affinity for phosphatidylserine and an antibody having an affinity for PSMA, to form a complex 2 of the substance having an affinity for phosphatidylserine, the extracellular vesicle having phosphatidylserine and PSMA, and the antibody having an affinity for a prostate specific membrane antigen, and
   (iii) a step of measuring an amount of the complex 2 obtained in the step (ii) to measure an amount of the extracellular vesicle having phosphatidylserine and PSMA.
[6] The method according to [5], in which in the step (i), the substance having an affinity for phosphatidylserine is bound to an insoluble carrier.
[7] The method according to [5] or [6], in which the step (i) includes the following steps;
   (i-1) a step of bringing the biological specimen into contact with a substance having an affinity for phosphatidylserine to form a complex 1 of an extracellular vesicle having phosphatidylserine in the biological specimen and the substance having an affinity for phosphatidylserine,
   (i-2) a step of separating and acquiring the complex 1 obtained in the step (i-1) from the biological specimen, and
   (i-3) a step of separating the extracellular vesicle having phosphatidylserine from the complex 1 obtained in the step (i-2) and acquiring the extracellular vesicle having phosphatidylserine.
[8] The method according to any one of [3] to [7], in which the substance having an affinity for phosphatidylserine is a T-cell immunoglobulin-mucin-domain containing protein.
[9] The method according to [8], in which the T-cell immunoglobulin-mucin-domain containing protein is a T-cell immunoglobulin-mucin-domain containing protein 1 or a T-cell immunoglobulin-mucin-domain containing protein 4.
[10] The method according to any one of [1] to [9], in which the biological specimen is a blood specimen.
[11] An examination kit for assisting diagnosis of metastatic CRPC, the kit comprising a substance having an affinity for phosphatidylserine and an antibody having an affinity for PSMA, which are used for measuring an extracellular vesicle having phosphatidylserine and PSMA.
[12] The kit according to [11], further comprising an insoluble carrier on which a substance having an affinity for phosphatidylserine is immobilized, where the substance is used for acquiring an extracellular vesicle having phosphatidylserine.
[13] A diagnostic biomarker for metastatic CRPC, the diagnostic biomarker comprising an extracellular vesicle having phosphatidylserine and PSMA.
[14] A device for assisting diagnosis of metastatic CRPC, the device comprising a measurement unit that measures an amount of an extracellular vesicle having phosphatidylserine and PSMA in a biological specimen derived from a subject.
[15] The device according to [14], further comprising a determination unit that determines whether or not a measured value obtained by the measurement in the measurement unit is equal to or larger than a reference value.

In consideration of the above circumstances, the inventors of the present invention have found that an extracellular vesicle having phosphatidylserine and PSMA (hereinafter, may be abbreviated as a "PS-PSMA extracellular vesicle"), which are present in the blood, can be a biomarker for diagnosing metastatic CRPC and have completed the present invention.

According to the present invention, it is possible to specifically diagnose metastatic CRPC by distinguishing it from non-metastatic CRPC, prostate cancer other than the CRPC, benign prostatic hyperplasia, and a healthy subject.

### BRIEF DESCRIPTION OF THE DRAWINGS

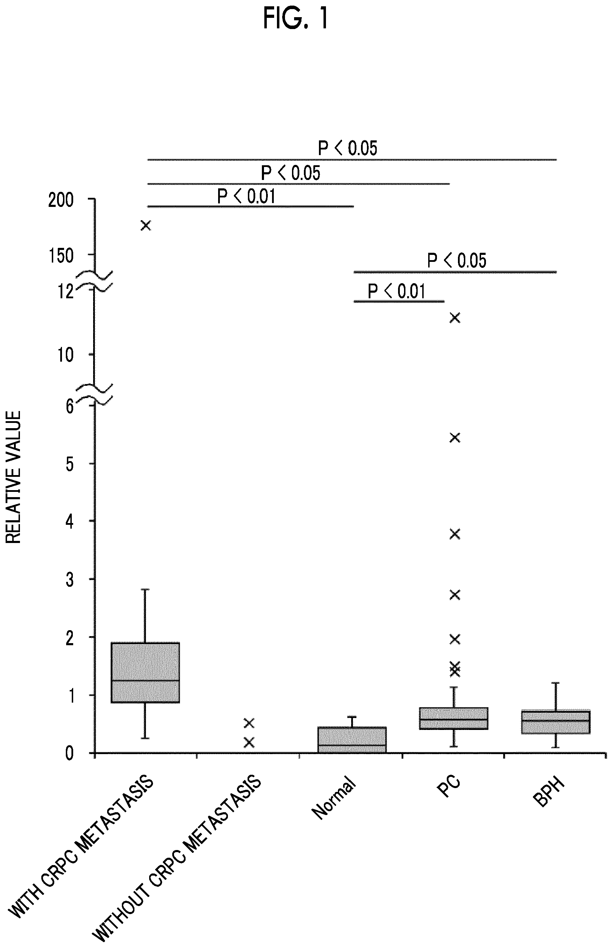

Fig. 1 is a graph showing relative values of measured values obtained in Example 1, which are obtained by using biological specimens derived from individual disease patients or a healthy subject, where the relative values are values with respect to an average value of measured values obtained in Example 1 by using a biological specimen derived from a CRPC patient 5, the average value being set to 1.

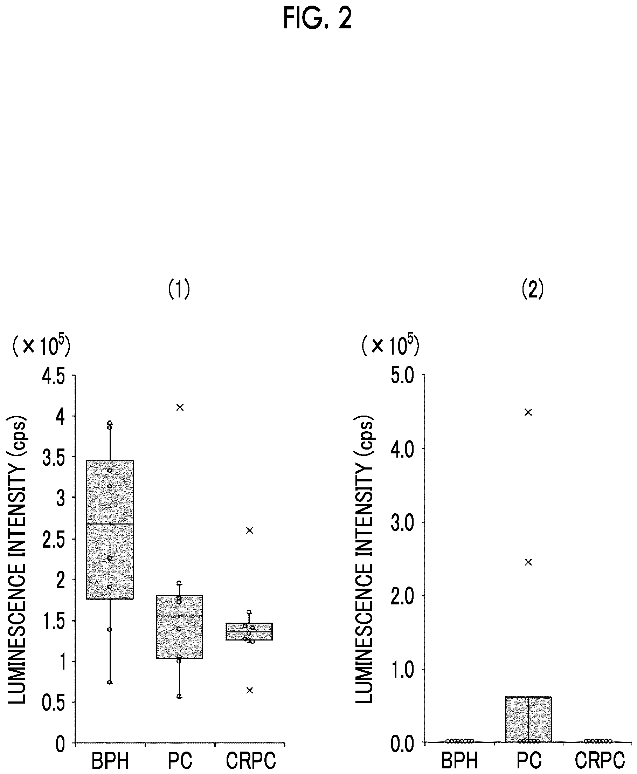

Fig. 2 are graphs showing measured values obtained in Comparative Example 1, which are obtained by using biological specimens derived from individual disease patients or a healthy subject. (1) of Fig. 2 is a graph showing results of carrying out measurement with a measurement system using an immobilized anti-PSMA antibody-labeled anti-CD9 antibody. (2) of Fig. 2 is a graph showing results of carrying out measurement with a measurement system with an immobilized anti-CD9 antibody-labeled anti-PSMA antibody.

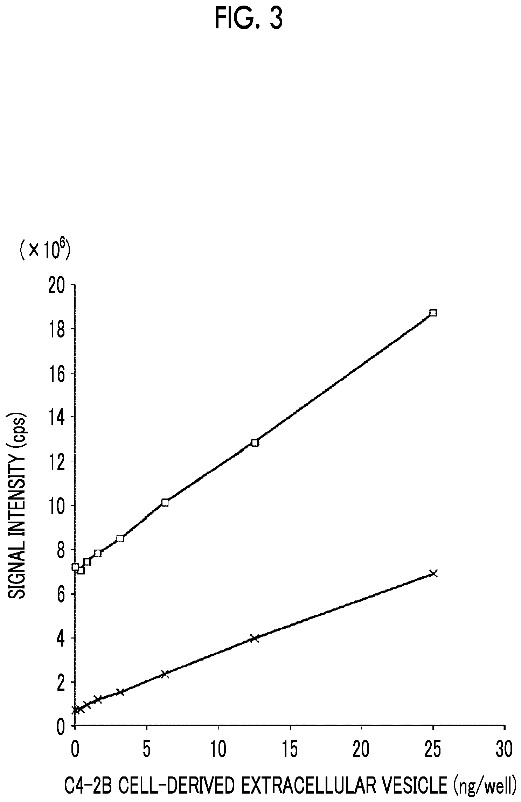

Fig. 3 is a graph showing results of measuring a PS-PSMA extracellular vesicle obtained in Example 2, which is obtained by using, as a specimen, a specimen containing a PS extracellular vesicle obtained by being separated from serum using Tim4 beads, or a serum containing an extracellular vesicle.

### DESCRIPTION OF THE PREFERRED EMBODIMENTS

### <1. Metastatic castration resistant prostate cancer>

"Metastasis" of cancer generally refers to that cancer cells migrate from the primary site to another organ in the body where they proliferate again.

In the present invention, the metastatic castration resistant prostate cancer refers to castration resistant prostate cancer (CRPC) that is metastatic. It includes the condition of a CRPC patient who already has metastasis, and the condition where castration resistant prostate cancer has acquired metastasis.

### <2. Extracellular vesicle having phosphatidylserine>

Examples of the extracellular vesicle having phosphatidylserine according to the embodiment of the present invention include small membrane vesicles derived from cells, the small membrane vesicles being composed of a lipid bilayer membrane and having phosphatidylserine on the membrane surface thereof. Examples of the diameter of the vesicle include generally 20 nm to 1,000 nm, preferably 50 to 800 nM, more preferably 50 nm to 500 nm, and particularly preferably 50 nm to 200 nm. Examples of the extracellular vesicle include those classified in various ways according to the generation origin thereof and the size of the small membrane vesicle, as described in Nature Reviews Immunology 9, 581-593 (August 2009); "Journal of Japan Society for the Study of Obesity" Vol. 13 No. 2, 2007, Topics Naoto Aoki et al.; and the like. Specific examples thereof include an exosome, a microvesicle, an ectosome, a membrane particle, an exosome-like vesicle, an apoptotic body, and an adiposome.

The exosome is a small membrane vesicle derived from the late endosome, which is composed of a lipid bilayer membrane and has phosphatidylserine on the membrane surface thereof. The diameter of the exosome is generally 50 nm to 200 nm, preferably 50 nm to 150 nm, and more preferably 50 nm to 100 nm. It is known that the exosome contains proteins such as tetraspanins such as CD63 and CD9, Alix, TSG101, Lamp-1, and Flotillin on the membrane surface thereof.

The microvesicle is a small membrane vesicle derived from the cell membrane, which is composed of a lipid bilayer membrane and has phosphatidylserine on the membrane surface thereof. The diameter of the microvesicle is generally 100 nm to 1,000 nm, preferably 100 nm to 800 nm, and more preferably 100 nm to 500 nm. It is known that the microvesicle contains proteins such as integrin, selectin, and a CD40 ligand on the membrane surface thereof.

The ectosome is a small membrane vesicle derived from the cell membrane, which is composed of a lipid bilayer membrane and has phosphatidylserine on the membrane surface thereof. The diameter of the ectosome is generally 50 nm to 200 nm, preferably 50 nm to 150 nm, and more preferably 50 nm to 100 nm. It is known that the ectosome contains CR1 and proteolytic enzymes on the membrane surface thereof but does not contain CD63.

The membrane particle is a small membrane vesicle derived from the cell membrane, which is composed of a lipid bilayer membrane and has phosphatidylserine on the membrane surface thereof. The diameter of the membrane particle is generally 50 nm to 80 nm. It is known that the membrane particle contains CD133 on the surface of the membrane but does not contain CD63.

The exosome-like vesicle is a small membrane vesicle derived from the early endosome, which is composed of a lipid bilayer membrane and has phosphatidylserine on the membrane surface thereof. The diameter of the exosome-like vesicle is generally 20 nm to 50 nm. It is known that the exosome-like vesicle contains tumor necrosis factor receptor 1 (TNFRI) on the membrane surface thereof.

The apoptotic body is a small membrane vesicle derived from the apoptotic cell, which is composed of a lipid bilayer membrane and has phosphatidylserine on the membrane surface thereof. The diameter of the apoptotic body is generally 50 nm to 500 nm, preferably 50 nm to 300 nm, and more preferably 50 nm to 200 nm. It is known that the apoptotic body contains histones on the membrane surface thereof.

The adiposome is a small membrane vesicle derived from the adipocyte, which is composed of a lipid bilayer membrane and has phosphatidylserine on the membrane surface thereof. The diameter of the adiposome is generally 100 nm to 1,000 nm, preferably 100 nm to 800 nm, and more preferably 100 nm to 500 nm. It is known that the adiposome contains milk fat globule-EGF factor 8 (MFG-E8).

the extracellular vesicle having phosphatidylserine is preferably exosomes or microvesicles, and more preferably exosomes.

Hereinafter, the "extracellular vesicle having phosphatidylserine" according to the embodiment of the present invention may be abbreviated as "PS extracellular vesicle".

The "extracellular vesicle having phosphatidylserine and PSMA" according to the embodiment of the present invention (hereinafter, may be abbreviated as the "PS-PSMA extracellular vesicle") is the above-described PS extracellular vesicle according to the embodiment of the present invention and the extracellular vesicle having PSMA on the membrane surface thereof.

### <3. Substance having affinity for phosphatidylserine>

It suffices that a "substance having an affinity for phosphatidylserine" according to the embodiment of the present invention has an affinity for phosphatidylserine in the presence of calcium ions and that it binds to phosphatidylserine present (exposed) on the membrane surface of the PS extracellular vesicle according to the embodiment of the present invention. Examples thereof include Annexin V, milk fat globule-EGF factor 8 (MFG-E8), and T-cell immunoglobulin-mucin-domain containing protein. It is preferably a T-cell immunoglobulin-mucin-domain containing protein (hereinafter, may be abbreviated as a "Tim protein").

The "substance having an affinity for phosphatidylserine" according to the embodiment of the present invention may be abbreviated as the "PS-affinitive substance" below.

### [Tim protein]

It suffices that the Tim protein according to the embodiment of the present invention has an affinity for phosphatidylserine in the presence of calcium ions and binds to the PS extracellular vesicle according to the embodiment of the present invention, and it is preferably an animal-derived Tim protein. Among the above, it is preferably a Tim protein possessed by a human.

More specifically, it suffices that the Tim protein is at least a Tim protein having an amino acid sequence of the binding domain (the IgV domain) to phosphatidylserine, where it may be one having an amino acid sequence of the full length of the Tim protein or may be a part of the Tim protein.

Examples of such a Tim protein according to the embodiment of the present invention include T-cell immunoglobulin-mucin-domain containing protein 1 (hereinafter abbreviated as "Tim1"), T-cell immunoglobulin-mucin-domain containing protein 3 (hereinafter abbreviated as "Tim3"), and T-cell immunoglobulin-mucin-domain containing protein 4 (hereinafter abbreviated as "Tim4"). Tim1 or Tim4 are preferable, and Tim4 is more preferable.

As an example, the amino acid sequence of the Tim protein according to the embodiment of the present invention is described in WO2016-088689A.

As the Tim protein according to the embodiment of the present invention, a commercially available product can be used. Further, it can also be obtained by a general chemical synthesis method or a genetic recombination technique as described in WO2016-088689A.

### <4. Method of assisting diagnosis of metastatic CRPC according to embodiment of present invention>

A method of assisting the diagnosis of metastatic CRPC of the present invention (hereinafter, may be abbreviated as an "assisting method of the present invention") includes;
(A) measuring an amount of a PS-PSMA extracellular vesicle in a biological specimen derived from a subject, and
(B) determining whether or not the subject has metastatic CRPC by using the amount of the PS-PSMA extracellular vesicles as an indicator.

The assisting method according to the embodiment of the present invention can be used as a method of assisting the diagnosis of metastatic CRPC by a doctor or the like. In addition, the assisting method of the present invention is carried out in vitro.

Examples of the "amount" of the PS-PSMA extracellular vesicle and the "amount" of the complex 2 in the assisting method of the present invention include values and concentrations in terms of volume or mass. In addition, they may be any one of a luminescence amount, absorbance, fluorescence intensity, turbidity, a transmitted light amount, a peak surface area, reflectance, and a refractive index, having a correlation with the "amount" of PS-PSMA extracellular vesicles and the "amount" of the complex 2, and a measured value of the amount of change in the value thereof or the like, as well as the relative value of the value calculated therefrom or the like.

In the assisting method of the present invention, the above (A) of the step of "measuring an amount of a PS-PSMA extracellular vesicle in a biological specimen derived from a subject" may be hereinafter abbreviated as the "measurement step according to the embodiment of the present invention".

In addition, the above (B) of the step of "determining whether or not the subject has metastatic CRPC by using the amount of the PS-PSMA extracellular vesicles as an indicator" may be hereinafter abbreviated as the "determination step according to the embodiment of the present invention".

### [Biological specimen]

It suffices that the biological specimen according to the embodiment of the present invention is derived from a human, and examples thereof include serum, plasma, whole blood, urine, semen, a bladder wash, tissue extract, prostate tissue section, and a prostate tissue biopsy sample, as well as those prepared therefrom. It is preferably a blood-derived specimen such as plasma, serum, or whole blood, and more preferably plasma or serum. It is particularly preferably serum.

The biological specimen according to the embodiment of the present invention may be, for example, those directly collected from a human or those that have undergone a pretreatment such as recovery, concentration, purification, isolation, dilution with a buffer solution, or filtration sterilization. These pretreatments may be appropriately carried out according to a conventional method that is used in the related field.

The method of obtaining (collecting) the biological specimen according to the embodiment of the present invention from a subject is not particularly limited, and it may be carried out, for example, by obtaining (collecting) the specimen from the subject based on a method known per se.

### <(A) Measurement step according to present invention>

Specifically, the measurement step according to the embodiment of the present invention includes, for example, the following steps.
(A-1) a step of obtaining an extracellular vesicle from a biological specimen derived from a subject,
(A-2) a step of bringing the extracellular vesicle obtained in the step (A-1) into contact with a PS-affinitive substance and an antibody having an affinity for PSMA, to form a complex 2 of, the PS-affinitive substance, the PS-PSMA extracellular vesicle, and the antibody having an affinity for PSMA (hereinafter, may be abbreviated as a "complex 2 formation step", and
(A-3) a step of measuring an amount of the complex 2 obtained in the step (A-2) to measure an amount of the PS-PSMA extracellular vesicle (hereinafter, may be abbreviated as a "measurement step").

### • (A-1) Step of acquiring extracellular vesicle from biological specimen derived from subject

It suffices that a method of obtaining an extracellular vesicle from a biological specimen according to step (A-1) is carried out by isolating extracellular vesicles according to a conventional method, which is not particularly limited.

Examples thereof include an affinity method (a PS affinity method using a phosphatidylserine binding molecule, or the like), a fractional centrifugation method (an ultracentrifugation method such as a pellet down method, a sucrose cushioning method, a density gradient centrifugation method, or the like), an immunoprecipitation method, a chromatography method (an ion exchange chromatography method, a gel permeation chromatography method, or the like), a density gradient method (a sucrose density gradient method or the like), an electrophoresis method (an organelle electrophoresis method or the like), a magnetic separation method (a magnetic activated cell sorting (MACS)) method), an ultrafiltration concentration method (a nanomembrane ultrafiltration concentration method or the like), a percoll gradient isolation method, a method using a microfluidic device, and a PEG precipitation method.

It is preferably an affinity method of obtaining extracellular vesicles with a high degree of purification or a fractional centrifugation method by which recovery can be carried out theoretically unbiasedly, more preferably an affinity method or an ultracentrifugation method, and particularly preferably an affinity method. Among the affinity methods, it is preferably a PS affinity method. The affinity method and the fractional centrifugation method may be carried out according to, for example, the methods described in WO2016-088689A.

One kind of this isolation method may be used, or two or more kinds thereof may be combined. Further, the isolation by one isolation method may be repeated twice or more.

The method of acquiring extracellular vesicle by using the PS-affinitive substance according to the embodiment of the present invention by the PS affinity method is carried out by bringing the biological specimen into contact with the PS-affinitive substance to form the complex 1 of the PS extracellular vesicle in the biological specimen and the PS-affinitive substance and subsequently separating the PS extracellular vesicle from the complex 1 to obtain the PS extracellular vesicle.

The preferred method of the step (A-1) by the PS affinity method specifically includes the following steps of (A-1-1) to (A-1-3).
(A-1-1) a step of bringing a biological specimen into contact with a PS-affinitive substance to form the complex 1 of the PS extracellular vesicle in the biological specimen and the PS-affinitive substance (hereinafter, may be abbreviated as a "complex 1 formation step"),
(A-1-2) a step of separating and acquiring the complex 1 obtained in the step of (A-1-1) from the biological specimen (hereinafter, may be abbreviated as a "complex 1 separation step"), and
(A-1-3) a step of separating the PS extracellular vesicle from the complex 1 acquired in the step (A-1-2) and acquiring the PS extracellular vesicle (hereinafter, may be abbreviated as an "acquisition step").

The extracellular vesicle obtained according to the PS affinity method is the PS extracellular vesicle.

### - (A-1-1) Complex 1 formation step -

The complex 1 formation step of (A-1-1) is a "step of bringing a biological specimen into contact with a PS-affinitive substance to form the complex 1 of the PS extracellular vesicle in the biological specimen and the PS-affinitive substance".

The biological specimen that is used in this step is as described above. The same applies to specific examples, preferred examples, and the like thereof.

In addition, specific examples of the PS-affinitive substance that is used in this step are as described in the section of "<3. Substance having affinity for phosphatidylserine>" described above. The same applies to specific examples, preferred examples, and the like thereof.

### • Method of immobilizing PS-affinitive substance on insoluble carrier

The PS-affinitive substance that is used in the complex 1 formation step is preferably a PS-affinitive substance bound to an insoluble carrier. In such a case, the complex 1 of the PS extracellular vesicle and the PS-affinitive substance can be separated from a reaction solution by a known B/F separation method in a complex 1 separation step of (A-1-2) described later.

An example of a method of immobilizing a PS-affinitive substance on an insoluble carrier will be described below; however, the method may be carried out, for example, according to the method described in WO2016-088689A.

As the insoluble carrier for immobilizing the PS-affinitive substance, any insoluble carrier can be used as long as it is an insoluble carrier that is used in a general immunological measuring method. Examples thereof include those prepared by using the following materials: organic substances such as polystyrene, polyacrylic acid, polymethacrylic acid, methyl polymethacrylate, polyacrylamide, polyglycidyl methacrylate, polypropylene, polyolefin, polyimide, polyurethane, polyester, polyvinyl chloride, polyethylene, polychlorocarbonate, a silicone resin, silicone rubber, agarose, dextran, and an ethylene-maleic acid anhydride copolymer; inorganic substances such as glass, silicon oxide, diatomite, porous glass, frosted glass, alumina, silica gel, and a metal oxide; magnetic substances such as iron, cobalt, nickel, magnetite, and chromate; and an alloy of these magnetic substances. Further, these carriers can be used in various forms such as a microplate, a tube, a disc-shaped piece, and a particle (a bead).

The insoluble carrier that is used in the complex 1 formation step of the step of (A-1-1) is preferably a particle (a bead). The size of the particle is not particularly limited; however, examples thereof include generally 10 nm to 100 µm and preferably 100 nm to 10 µm.

Examples of the method of binding the PS-affinitive substance to the insoluble carrier include a method known per se of binding a protein to a carrier. Specific examples thereof include a method of carrying out binding by affinity binding, a method of carrying out binding by chemical bonding (for example, the method disclosed in JP3269554B or WO2012/039395A), and a method of carrying out binding by physical adsorption (for example, the method disclosed in JP1993-41946A (JP-H5-41946A)), where a method of carrying out binding by physical adsorption or a method of carrying out binding by affinity binding is preferable. A method of carrying out binding by physical adsorption is simple and thus is more preferable.

In the method of binding the PS-affinitive substance to the insoluble carrier according to the embodiment of the present invention by physical adsorption, it suffices that the PS-affinitive substance is brought into contact with the insoluble carrier under the conditions in which the PS-affinitive substance is bound to the insoluble carrier according to a method known per se.

The amount of the PS-affinitive substance according to the embodiment of the present invention to be bound to the insoluble carrier is generally 0.1 µg to 50 µg, preferably 0.1 µg to 30 µg, and more preferably 0.1 µg to 20 µg, with respect to 1 mg of the insoluble carrier, for example, in a case where the insoluble carrier is a particle (a bead). In addition, in a case where the insoluble carrier is a microplate, it is generally 0.1 µg to 10 µg, preferably 0.1 µg to 5 µg, and more preferably 0.1 µg to 2 µg, with respect to one well of the microplate.

The physical adsorption between the PS-affinitive substance and the insoluble carrier is carried out by bringing a solution containing the PS-affinitive substance into contact with the insoluble carrier.

Regarding the solution containing the PS-affinitive substance, it suffices that the solution that dissolves the substance is any solution that dissolves the PS-affinitive substance in a stable state, and examples thereof include purified water, for example, a buffer solution having a buffering action at a pH of 6.0 to 9.8 and preferably at a pH of 7.0 to 9.6 (for example, a Good buffer solution such as MOPS, a carbonate buffer solution, PBS, TBS, TBS-T, HBS, or the like). In addition, the buffering agent concentration in these buffer solutions is appropriately selected generally from a range of 5 to 100 mM and preferably 10 to 100 mM, and the concentration in a case where NaCl is added is appropriately selected generally from a range of 100 to 200 mM and preferably from a range of 140 to 160 mM. In addition, the solution that dissolves the PS-affinitive substance may contain, for example, saccharides, salts such as NaCl, a surfactant such as Tween 20, a preservative, and a protein, as long as the amounts thereof do not interfere with the binding between the PS-affinitive substance and the insoluble carrier.

The Tim protein is preferable as the PS-affinitive substance that is immobilized on the insoluble carrier. Tim4 is more preferable. Hereinafter, the insoluble carrier on which the Tim protein is immobilized may be abbreviated as the "Tim carrier". Hereinafter, the insoluble carrier on which the Tim4 protein is immobilized may be abbreviated as the "Tim4 carrier".

Specific examples of the method of binding the PS-affinitive substance to the insoluble carrier by physical adsorption include the following method.

First, in a case where the insoluble carrier is a particle (a bead), 1 mg of the bead carrier is brought into contact with a solution containing the PS-affinitive substance of generally 0.1 µg to 50 µg and preferably 1 µg to 50 µg with generally 50 µL to 300 µL, preferably 50 µL to 200 µL, and more preferably 50 µL to 100 µL, and the reaction is carried out generally at 2°C to 37°C and preferably 4°C to 11°C, generally for 0.5 to 48 hours and preferably 0.5 to 24 hours.

In a case where the insoluble carrier is a microplate, one well of the microplate is brought into contact with a solution containing the PS-affinitive substance of generally 0.1 µg to 10 µg, preferably 0.2 µg to 5 µg, and more preferably 0.5 µg to 2 µg, with generally 50 µL to 300 µL, preferably 50 µL to 200 µL, and more preferably 50 µL to 100 µL, and reaction is carried out generally at 2°C to 37°C and preferably 4°C to 11°C, generally for 4 to 48 hours and preferably 12 to 24 hours.

The insoluble carrier on which the PS-affinitive substance is immobilized, which has been obtained as described above, may be subjected to a blocking treatment that is generally carried out in the related field.

In addition, the insoluble carrier on which the PS-affinitive substance according to the embodiment of the present invention is immobilized, which has been obtained as described above, may be as necessary subjected to a purification treatment that is generally carried out in the related field. It suffices that with the purification treatment, impurities that have adhered to the surface of the carrier can be removed. Examples of the purification treatment include a method of washing the insoluble carrier with a washing solution (hereinafter, may be abbreviated as a "washing operation"). The washing operation may be repeated several times as needed.

### • Complex 1 formation step

The complex 1 formation step of (A-1-1) is carried out in the presence of calcium ions in a case where the Tim protein is used as a PS-affinitive substance. That is, calcium ions are allowed to be present when the PS-affinitive substance is brought into contact with the extracellular vesicle in the biological specimen.

The concentration of calcium ions at the time of bringing the PS-affinitive substance into contact with the extracellular vesicle in the biological specimen is generally 0.5 mM to 100 mM, preferably 1.0 mM to 10 mM, and more preferably 2.0 mM to 5.0 mM. It is noted that after the complex 1 of the PS-affinitive substance and the PS extracellular vesicle in the biological specimen is formed and until an acquisition step (A-1-3) described later is carried out, that is, until carrying out the step of separating the complex 1, it goes without saying that calcium ions having the same concentration as described above are required in the solution containing the complex 1.

The origin of the calcium ions is not particularly limited, and examples thereof include calcium chloride, calcium hydroxide, calcium hydrogen carbonate, calcium iodide, calcium bromide, and calcium acetate. It is preferably calcium chloride, calcium hydrogen carbonate, or calcium iodide, and more preferably calcium chloride or calcium hydrogen carbonate.

In the method of allowing calcium ions to be present at the time of bringing the PS-affinitive substance into contact with the extracellular vesicle in the biological specimen, it suffices that the same calcium ions as described above are contained in the biological specimen or/and the solution containing the PS-affinitive substance so that the concentration of calcium ions at the time of bringing the PS-affinitive substance into contact with the extracellular vesicle in the biological specimen is within the above range. In addition, a solution containing calcium ions (hereinafter, may be abbreviated as a "solution containing calcium ions"), the biological specimen, and the solution containing the PS-affinitive substance may be mixed so that the concentration of calcium ions at the time of bringing the PS-affinitive substance into contact with the extracellular vesicle in the biological specimen is within the above range.

In the solution containing calcium ions according to the embodiment of the present invention, the solution that dissolves calcium ions may be any solution that does not interfere with the binding between the PS extracellular vesicle and the PS-affinitive substance, examples thereof include water and a buffer solution having a buffering action at pH 7.0 to pH 8.0 and preferably at pH 7.2 to pH 7.6 (for example, TBS, HBS, or the like). It is noted that the phosphate buffer is not preferable since it binds to calcium and causes precipitation. In addition, the concentration of the buffering agent in these buffer solutions is appropriately selected generally from a range of generally 5 mM to 50 mM and preferably 10 mM to 30 mM. The concentration of NaCl is appropriately selected generally from a range of 100 mM to 200 mM and preferably 140 mM to 160 mM.

The solution containing calcium ions according to the embodiment of the present invention may contain, for example, saccharides, salts such as NaCl, a surfactant, a preservative, and a protein such as BSA, as long as the amounts thereof do not interfere with the binding between the PS extracellular vesicle and the PS-affinitive substance. Examples of the surfactant include Tween 20, and the concentration of the surfactant in the solution containing calcium ions according to the embodiment of the present invention is generally 0.00001% to 0.2% and preferably 0.0005% to 0.1%.

In the complex 1 formation step, the amount of the biological specimen to be contacted with 1 µg of the PS-affinitive substance (which may be immobilized on the insoluble carrier) is generally 0.1 ml to 100 ml, preferably 0.1 ml to 10 ml, and more preferably 0.1 ml to 1.0 ml. The temperature at which the biological specimen is brought into contact with the PS-affinitive substance (which may be immobilized on the insoluble carrier) is generally 2°C to 37°C, preferably 4°C to 37°C, and more preferably 4°C to 30°C. The time of contact between the biological specimen and the PS-affinitive substance is generally 0.5 to 24 hours, preferably 0.5 to 8 hours, and more preferably 0.5 to 4 hours.

In a case where the PS-affinitive substance that has been bound to the insoluble carrier is used in the complex 1 formation step, the amount of the carrier is generally 0.1 mg to 20 mg, preferably 0.3 mg to 10 mg, and more preferably 0.5 mg to 6.0 mg with respect to 1 mL of the solution at the time of forming the complex 1.

The complex 1 formation step may be carried out by, for example, the following method.

That is, an insoluble carrier on which the PS-affinitive substance is immobilized, where the amount of the PS-affinitive substance is generally 0.1 mg to 20 mg, preferably 0.3 mg to 10 mg, and more preferably 0.5 mg to 6.0 mg per 1 mL of a solution after mixing, the solution containing calcium ions according to the embodiment of the present invention, the amount of which is such that the concentration of calcium ions in the solution after mixing is generally 0.5 mM to 100 mM, preferably 1.0 mM to 10 mM, and more preferably 2.0 mM to 5.0 mM, and a biological specimen of generally 0.1 ml to 100 ml, preferably 0.1 ml to 10 ml, and more preferably 0.1 ml to 1.0 ml per 1 mg of the insoluble carrier on which a PS-affinitive substance is immobilized are brought into contact and mixed with each other generally at 4.0°C to 37°C, preferably 4.0°C to 25°C, and more preferably 4.0°C to 11°C, generally for 0.5 to 24 hours, preferably 0.5 to 8.0 hours, and more preferably 0.5 to 4.0 hours, whereby a complex (the complex 1) of the PS-affinitive substance bound to the carrier and the PS extracellular vesicle in the biological specimen is formed.

### - (A-1-2) Complex 1 separation step -

The complex 1 separation step of (A-1-2) is a step of separating and obtaining the complex 1 obtained in the step of (A-1-1) from the biological specimen.

It is noted that the biological specimen may be the biological specimen itself or may be a solution that contains the solution containing calcium ions according to the embodiment of the present invention.

The complex 1 separation step may be any method as long as the complex 1 and the biological specimen can be separated to acquire the complex 1, where such a method may be carried out using the known B/F separation, and specific examples thereof include the following methods.
(i) In a case where the carrier of the insoluble carrier on which the PS-affinitive substance is immobilized is a magnetic carrier: a method of installing a container containing the complex 1 obtained in the complex 1 formation step on a magnet stand as necessary, collecting the complex 1 on the tube wall by using magnetic force, and removing a supernatant to separate the complex 1.
(ii) In a case where the carrier of the insoluble carrier on which the PS-affinitive substance is immobilized is has a bead shape: a method of subjecting a container containing the complex 1 obtained in the complex 1 formation step to a centrifugation treatment to collect the complex 1 as a precipitate and subsequently removing a supernatant to separated the complex 1.
(iii) In a case where the carrier of the insoluble carrier on which the PS-affinitive substance is immobilized is a plate or the like: a method of removing only a solution containing the biological specimen to separate the complex 1.
(iv) a method of separating the complex 1 and a solution containing the biological specimen by filtration.

After separating the complex 1 and the solution containing the biological specimen in this way, the separated complex 1 may be obtained (recovered) by a method known per se.

Specific examples of the complex 1 separation step include the following method.

In a case where a magnetic carrier is used as the insoluble carrier, a container subjected to the complex 1 formation step is installed on a magnet stand as necessary, and the complex 1 obtained on the tube wall is collected by using magnetic force, and the specimen supernatant is removed.

After carrying out the complex formation step of (A-1-1) and the complex separation step of (A-1-2), the obtained complex 1 may be washed as necessary using a calcium ion-containing washing solution (hereinafter, may be abbreviated as "washing operation"). The washing operation makes it possible to remove impurities in the biological specimen, such as cell-derived components which have adhered to the surface of the insoluble carrier on which the PS-affinitive substance is immobilized. As the washing method, in addition to using a calcium ion-containing washing solution, a washing method generally carried out in the related field can be used.

The calcium ion-containing washing solution that is used in the washing operation may be any solution as long as it contains calcium ions generally at 0.5 to 100 mM, preferably generally 1 to 10 mM, and more preferably generally 2 mM to 5 mM, and does not affect the binding between the PS extracellular vesicle and the PS-affinitive substance immobilized on the insoluble carrier. Examples thereof include buffer solutions which do not precipitate calcium (for example, TBS, TBS-T, and HBS) containing calcium ions generally at 0.5 mM to 100 mM, preferably generally 1 mM to 10 mM, and more preferably generally 2 mM to 5 mM and having a buffering action at pH 7.0 to pH 8.0 and preferably pH 7.2 to pH 7.6. It is noted that the phosphate buffer is not preferable since it binds to calcium and causes precipitation. In addition, the buffering agent concentration in these buffer solutions is appropriately selected generally from a range of 5 mM to 50 mM and preferably 10 mM to 30 mM, and the concentration of NaCl is appropriately selected generally from a range of 100 mM to 200 mM and preferably from a range of 140 mM to 160 mM. This solution may contain, for example, saccharides, salts such as NaCl, a surfactant, a preservative, and a protein, as long as the amounts thereof do not interfere with the binding between the PS extracellular vesicle and the PS-affinitive substance immobilized on the insoluble carrier. Examples of the surfactant include tween 20 (manufactured by FUJIFTLM Wako Pure Chemical Corporation), and the concentration of the surfactant in the washing solution is generally 0.00001% to 0.2% and preferably 0.0005% to 0.1%.

A specific example of the washing operation will be described by taking, as an example, a washing operation using magnetic particles as an insoluble carrier for immobilizing the PS-affinitive substance.

That is, the calcium ion-containing washing solution according to the embodiment of the present invention is added in a container containing the complex 1 obtained by the complex 1 separation step, and the resultant mixture is stirred. Then, the container is installed on a magnet stand, the complex 1 is collected on the tube wall by using magnetic force, and then the solution in the container is discarded. These washing operations may be repeated several times as needed.

### - (A-1-3) Acquisition step -

The acquisition step of (A-1-3) is a step of carrying out the complex 1 formation step of (A-1-1) and the complex 1 separation step of (A-1-2), and as necessary, a washing operation, and subsequently separating the PS extracellular vesicle from the acquired complex 1 to acquire the PS extracellular vesicle according to the embodiment of the present invention.

Examples of the acquisition step include a method of using a protein denaturing agent and a method of reducing the concentration of calcium ions, where a method of reducing the concentration of calcium ions is preferable since intact PS extracellular vesicles can be obtained.

The method of using a protein denaturing agent is carried out by carrying out the complex 1 formation step and the complex 1 separation step and as necessary, after carrying out a washing operation, subsequently causing a protein denaturing agent to act on the obtained complex 1 according to the embodiment of the present invention to denature the PS-affinitive substance according to the embodiment of the present invention in the complex 1, and separating the PS extracellular vesicles from the complex 1. This makes it possible to acquire the PS extracellular vesicle according to the embodiment of the present invention. In the method of using a protein denaturing agent, the concentration to be used, the using conditions, or the like of the protein denaturing agent are generally set according to the method that is used in the related field.

The protein denaturing agent may be any denaturing agent that is generally used as a compound that denatures proteins in the related field. Examples thereof include anionic surfactants such as sodium dodecyl sulfate (SDS) and N-lauroyl sarcosine; amphoteric surfactants such as 3-(3-cholamidopropyl)dimethylammonio-1-propanesulfonate hydrate (CHAPS) and N-dodecyl-N,N-dimethyl-3-ammonio-1-propanesulfonate (Zwittergent 3-12); nonionic surfactants such as Brij 35 (manufactured by Takara Bio Inc.), dodecyl-β-D-maltoside (n-dodecyl-β-D-maltoside), Nonidet P-40, octyl-P-D-glucoside, polyoxyethylene(10) octylphenyl ether (Triton X-100), and polyoxyethylene(20) sorbitan monolaurate (Tween 20); and chaotropic agents such as urea, formamide, and guanidine, where an anionic surfactant is preferable, and SDS is particularly preferable.

Examples of the method of reducing the concentration of calcium ions include a method of using a calcium ion chelating agent.

That is, it is a method of carrying out the complex 1 formation step and the complex 1 separation step, subsequently causing a calcium ion chelating agent to act on calcium ions bound to the complexes 1 and calcium ions brought in from a solution containing the complexes 1, and then reducing the (effective) concentration of the calcium ions (chelating the calcium ions) bound to the complexes 1 and the calcium ions brought in from the solution containing the complexes 1, thereby separating the PS extracellular vesicles from the complexes 1.

The calcium ion chelating agent that is used in this method may be any chelating agent that is capable of chelating calcium ions. Examples thereof include ethylenediaminetetraacetic acid (EDTA), nitrilotriacetic acid (NTA), diethylenetriaminetetraacetic acid (DTPA), L-glutamic acid diacetic acid (GLDA), hydroxyethylethylenediaminetriacetic acid (HEDTA), ethylene glycolbis(β-aminoethyl ether)-N,N,N,N,-tetraacetic acid (GEDTA), triethylenetetramine-N,N,N',N",N‴,N‴-hexacetic acid (TTHA), 2-hydroxyethyliminodiacetic acid (HIDA), N,N-bis(2-hydroxyethyl)glycine (DHEG), and trans-1,2-diaminocyclohexane-N,N,N',N'-tetraacetic acid, monohydrate (CyDTA), where EDTA, GEDTA, or CyDTA is preferable.

The calcium ion chelating agent is generally used as a form of a solution. A solution that dissolves the calcium ion chelating agent may be any solution that dissolves the calcium ion chelating agent, and examples thereof include purified water and a buffer solution. The buffer solution is preferably a buffer solution having a buffering action generally at pH 7.0 to pH 8.0 and preferably pH 7.2 to pH 7.6 (for example, PBS, TBS, HBS, or the like). In addition, the buffering agent concentration in these buffer solutions is appropriately selected generally from a range of 5 Mm to 50 Mm and preferably 10 Mm to 30 Mm, and the concentration of NaCl is appropriately selected generally from a range of 100 Mm to 200 Mm and preferably from a range of 140 Mm to 160 Mm. The calcium ion chelating agent-containing solution may contain, for example, saccharides, salts such as NaCl, a preservative, and protein.

The concentration of the calcium ion chelating agent in the calcium ion chelating agent-containing solution is generally 0.5 mM to 500 mM, preferably 0.5 mM to 100 mM, and more preferably 0.5 mM to 50 mM. The pH of the calcium ion chelating agent-containing solution is generally pH 6.0 to pH 9.0, preferably pH 7.0 to pH 8.0, and more preferably pH 7.2 to pH 7.6.

In order to cause the calcium ion chelating agent to act on the calcium ion bound to the complex 1, the calcium ion chelating agent-containing solution is brought into contact with the pellet-shaped complex 1, and the calcium ion bound to the complex 1 is reacted with the calcium ion chelating agent in the calcium ion chelating agent-containing solution.

For example, the contact between the calcium ion chelating agent-containing solution and the complex 1 can be carried out by, for example, a method of suspending the complex 1 in the above solution (in a case where the insoluble carrier of the insoluble carrier on which the PS-affinitive substance is immobilized is a bead, or the like), a method of immersing the complex 1 in the above solution (in a case where the insoluble carrier of the insoluble carrier on which the PS-affinitive substance is immobilized is a disc-shaped piece or a tube, or the like), or a method of adding the above solution to the complex 1 (in a case where the insoluble carrier of the insoluble carrier on which the PS-affinitive substance is immobilized is a microplate or a tube, or the like).

It suffices that the amount of the calcium ion chelating agent-containing solution to be brought into contact with the complex 1 is such an amount that the concentration of calcium ions in the solution after contact with the complex 1 is less than the effective concentration and the extracellular vesicle is separated from the complex 1.

The temperature and the time, at which the calcium ion chelating agent is allowed to act on (be brought into contact with) the complex 1, are generally 4.0°C to 37°C, preferably 10°C to 30°C, and more preferably 20°C to 30°C, generally for 1 to 0 minutes and preferably 5 to 15 minutes.

The acquisition step according to the embodiment of the present invention is as follows in a case of being described by taking the method of using a Tim carrier as an example.

That is, after carrying out the complex 1 separation step and the complex 1 formation step, and as necessary, after carrying out a washing operation, a solution containing a calcium ion chelating agent of which the concentration is generally 0.5 mM to 500 mM, preferably 0.5 mM to 100 mM, and more preferably 0.5 mM to 50 mM is added to the obtained complex 1, where the volume of the solution is generally 10 µL to 500 µL, preferably 20 µL to 200 µL µL, and more preferably 50 µL to 100 µL µL per 1 mg of the Tim carrier, the resultant mixture is reacted, while being stirred using a vortex mixer or the like, generally at 4.0°C to 37°C, preferably 10°C to 30°C, and more preferably 20°C to 30°C, generally for 1 to 30 minutes and preferably 5 to 15 minutes, and the extracellular vesicle (the PS extracellular vesicle) is separated from the complex 1.

As a result of carrying out the acquisition step of (A-1-3), the calcium ion chelating agent-containing solution brought into contact with (allowed to act on) the complex 1 contains the insoluble carrier on which the PS-affinitive substance is immobilized, and the extracellular vesicle separated (liberated) from the insoluble carrier (the complex 1) on which the PS-affinitive substance is immobilized. Accordingly, in a case where the carrier is removed from the solution and only the solution is recovered, a solution containing the PS extracellular vesicle can be obtained.

It is noted that in a case where the extracellular vesicle is acquired by the PS affinity method in the above (A-1), the acquired extracellular vesicle is substantially the PS extracellular vesicle.

### • (A-2) Complex 2 formation step

The step (A-2) is carried out by bringing the extracellular vesicle obtained in the step (A-1) into contact with a PS-affinitive substance and an antibody having an affinity for PSMA, to form the complex complex 2 of the PS-affinitive substance, the PS-PSMA extracellular vesicle, and the antibody having an affinity for PSMA.

### (1) Antibody having affinity for PSMA

It suffices that an antibody having an affinity for PSMA according to the embodiment of the present invention (hereinafter, may be abbreviated as an "anti-PSMA antibody") has an affinity for PSMA and has a property of binding to PSMA, and an antibody that specifically binds to PSMA is preferable. In addition, the anti-PSMA antibody may be a monoclonal antibody or a polyclonal antibody. A monoclonal antibody is preferable. It may be a commercially available product or an antibody prepared by a conventional method.

Further, the anti-PSA antibody may be Fab, Fab', F(ab')2, Fv, Fd, single chain Fv (scFv), disulfide bonded Fv (sdFv), VL, VH, a diabody ((VL-VH)2 or (VH-VL)2), a triabody (a trivalent antibody), a tetrabody (a tetravalent antibody), a minibody ((scFV-CH3)2), IgG-delta-CH2, scFv-Fc, an (scFv)2-Fc fragment, or the like thereof.

In addition, the preparation of these antibodies may be carried out, for example, according to the method described in "Immunoassay" (edited by the Immunochemical Measurement Study Group, Kodansha, 2014).

The origin of the anti-PSMA antibody is not particularly limited; however, the anti-PSMA antibody is an antibody that is derived from, for example, a rabbit, a rat, a mouse, sheep, a goat, or a horse, and has the properties described above.

The anti-PSMA antibody that is used in the complex 2 formation step may be immobilized on the insoluble carrier or may be labeled with a labeling substance or the like; however, it is preferably labeled with a labeling substance or the like. For example, in a case where an anti-PSMA antibody labeled with a labeling substance (a labeled anti-PSMA antibody) is used, the amount of the labeling substance of the labeled anti-PSMA antibody is measured, and based on this amount, the amount of the complex 2 may be determined.

As examples of the method of immobilizing the anti-PSMA antibody on an insoluble carrier and the insoluble carrier to be used, an insoluble carrier commonly that is used in the related field may be used, and the antibody may be immobilized according to a conventional method of carrying out immobilization on the insoluble carrier.

Examples of the labeling substance to used for labeling the anti-PSMA antibody include enzymes that are used in the general immunoassay, such as alkaline phosphatase, peroxidase (POD), microperoxidase, β-galactosidase, glucose oxidase, glucose-6-phosphate dehydrogenase, acetylcholinesterase, malate dehydrogenase, and luciferase; radioactive isotopes that are used in the radioimmunoassay (RIA), such as 99mTc, 1311, 1251, 14C, 3H, 32P, and 35S; fluorescent substances that are used in the fluoroimmunoassay (FIA), such as fluorescein, dansyl, fluorescamine, coumarin, naphthylamine, fluorescein isothiocyanate (FITC), rhodamine, rhodamine X isothiocyanate, sulforhodamine 101, lucifer yellow, acridine, acridine isothiocyanate, riboflavin, and derivatives thereof; luminescent substances such as luciferin, isoluminol, luminol, and a bis(2,4,6-trifluorophenyl)oxalate; substances having absorption in an ultraviolet region such as phenol, naphthol, anthracene, and derivatives thereof; labeling substances such as substances having properties as spin labeling agents represented by compounds having an oxyl group such as 4-amino-2,2,6,6-tetramethylpiperidine-1-oxyl, 3-amino-2,2,5,5-tetramethylpyrrolidine-1-oxyl, and 2,6-di-t-butyl-a-(3,5-di-t-butyl-4-oxo-2,5-cyclohexadien-1-ylidene)-p-tolyloxyl; HiLyte coloring agents such as HiLyte Fluor 647, HiLyte Fluor 488, HiLyte Fluor 555, HiLyte Fluor 680, and HiLyte Fluor 750 (all of which are trade names of HiLyte Bioscience, Inc.); Alexa coloring agents such as Alexa Fluor Dye 350, Alexa Fluor Dye 430, Alexa Fluor Dye 488, Alexa Fluor Dye 532, Alexa Fluor Dye 546, Alexa Fluor Dye 555, Alexa Fluor Dye 568, Alexa Fluor Dye 594, Alexa Fluor Dye 633, Alexa Fluor Dye 647, Alexa Fluor Dye 660, Alexa Fluor Dye 680, Alexa Fluor Dye 700, and Alexa Fluor Dye 750 (all of which are trade names of Molecular Probes, Inc.); CyDye coloring agents such as Cy3, Cy3.5, Cy5, Cy5.5, and Cy7 (all of which are trade names of Amersham Biosciences, Inc.); and coloring agents such as Coomassie Brilliant Blue R250 and Methyl Orange. Among them, enzymes such as peroxidase, microperoxidase, alkaline phosphatase, β-galactosidase, glucose oxidase, glucose-6-phosphate dehydrogenase, acetylcholinesterase, malate dehydrogenase, and luciferase are preferable, and alkaline phosphatase or peroxidase is more preferable.

In order to bind (label) the same labeling substance as described above to the anti-PSMA antibody, it is sufficient to appropriately use, for example, a known labeling method per se generally used in the immunoassay such as EIA, RIA, or FIA, which is known per se.

For example, the following method may be appropriately used: Enzyme Labeling Method, p.62, written by Eiji Ishikawa, Japan Scientific Societies Press, 1991; Medical Chemistry Experiment Course, Volume 8, supervised by Yuichi Yamamura, First edition, Nakayama Shoten Co., Ltd., 1971; Fluorescent Antibody with Illustrations, written by Akira Kawao, First edition, Soft Science Co., Ltd., 1983; Enzyme Immunoassay, Eiji Ishikawa, Tadashi Kawai, Kiyoshi Muroi, Second edition, Igaku-Shoin Ltd., 1982, or the like; or a method described in Molecular Cloning: A Laboratory Manual, Second Edition, J. Sambrook, E. F. Fritsch, and T. Maniatis, Cold Spring Harbor Laboratory Press, Cold Spring Harbor, or a conventional method using a reaction between avidin (or streptavidin) and biotin.

A commercially available kit with which the same labeling substance as described above is bound (labeled) to a protein may be used to carry out labeling of the anti-PSMA antibody according to the user's manual attached to the kit.

In addition, in a case of carrying out high performance liquid chromatography, capillary electrophoresis, or capillary chip electrophoresis, a separation improving substance such as a nucleic acid such as DNA or RNA may be bound in order to more clearly separate the antigen-antibody complex from the liberated labeled anti-PSMA antibody (JP3070418B, JP3531372B, or the like).

### (2) Extracellular vesicle

Regarding the extracellular vesicle obtained in the step (A-1) related to the complex 2 formation step, there are a case where it contains substantially only the PS extracellular vesicle and a case where it contains both the PS extracellular vesicle and an extracellular vesicle having no phosphatidylserine. The extracellular vesicle obtained by the PS affinity method in the step (A-1) is preferable since it consists of substantially only the PS extracellular vesicle.

In the following descriptions related to the complex 2 formation step, both of the above extracellular vesicles are included unless otherwise specified, in a case where "extracellular vesicle obtained in (A-1)" or "extracellular vesicle" is described.

### (3) PS-affinitive substance

The PS-affinitive substance that is used in the complex 2 formation step is as described in the section of "<3. Substance having affinity for phosphatidylserine>" described above. The same applies to specific examples, preferred examples, and the like thereof, and the Tim protein is preferable. Tim4 is more preferable.

It is noted that although the PS-affinitive substance according to the embodiment of the present invention may be immobilized on the insoluble carrier or may be labeled with a labeling substance, it is necessary to separate a liberated labeled anti-PSMA antibody from the complex 2 in a case where the anti-PSMA antibody labeled in the complex 2 formation step is used. In this case, the PS-affinitive substance described above is preferably a PS-affinitive substance immobilized on an insoluble carrier. This makes it possible to separate the liberated labeled anti-PSMA antibody from the complex 2 by the known B/F separation method after forming the complex 2.

Specific examples of the method of labeling the PS-affinitive substance and the labeling substance to be used therefor are respectively based on the above-described method of labeling the anti-PSMA antibody and the specific examples of the labeling substance.

The insoluble carrier that is used in the complex 2 formation step (A-2) is preferably a particle (a bead) or a microplate. In a case of a particle, the size thereof is not particularly limited; however, examples thereof include generally 10 nm to 100 µm and preferably 100 nm to 10 µm. In addition, in a case of a microplate, the number and the size of wells thereof are not particularly limited; however, the microplate includes those having generally 12 wells to 1,536 wells and preferably 96 wells to 384 wells.

A method of immobilizing the PS-affinitive substance on the insoluble carrier and preferred specific examples thereof are as described in the above-described section of "Method of immobilizing PS-affinitive substance on insoluble carrier" in the complex 1 formation step of (A-1-1) described above. The same applies to specific examples, preferred examples, and the like thereof, and a Tim carrier obtained by immobilizing the Tim protein on the insoluble carrier is preferable. A Tim4 carrier obtained by immobilizing Tim 4 on the insoluble carrier is more preferable.

### (4) Complex 2 formation step

In the complex 2 formation step, the extracellular vesicle obtained in the step (A-1) is brought into contact with the Tim protein in the presence of calcium ions. The concentration of calcium ions in the solution is generally 0.5 mM to 100 mM, preferably 1 mM to 50 mM, and more preferably 2 mM to 50 mM. It is noted that after the complex 2 according to the embodiment of the present invention is formed and until it is subjected to the step of measuring the amount of the complex 2, the calcium ions having the above-described concentration are required in the solution containing the complex 2.

Further, the origin of the calcium ions is not particularly limited, and specific examples thereof include the same one as the origin of the calcium ions in the step of (A-1-1).

Examples of the method of bringing the extracellular vesicle obtained in the step (A-1) into contact with the PS-affinitive substance in the presence of calcium ions include a method of mixing a solution containing calcium ions, a solution containing the extracellular vesicle, and a solution containing the PS-affinitive substance so that the concentrations of calcium ions at the time of bringing the extracellular vesicle into contact with the PS-affinitive substance is within the above range.

Regarding the above solution containing calcium ions, examples of a solution in which calcium ions are dissolved and the additive thereof are as described in the section of "(2) Complex 1 formation step" of the step (A-1), and the same applies to specific examples, preferred examples, and the like thereof.

Regarding the order of bringing the extracellular vesicle obtained in the step (A-1), the PS-affinitive substance, and the anti-PSMA antibody into contact with each other, they may be brought into contact at the same time or at different times; however, it is preferable that the extracellular vesicle is brought into contact with the PS-affinitive substance and then the anti-PSMA antibody is brought into contact therewith.

### (4-1) Reaction between extracellular vesicle and PS-affinitive substance

In a case where the PS-affinitive substance is immobilized on a bead, the amount of the solution containing the extracellular vesicle obtained in the step (A-1), which is reacted with the PS-affinitive substance, is generally 0.1 mL to 1,000 mL, preferably 0.1 mL to 500 mL, and more preferably 0.1 mL to 100 mL with respect to 1 mg of the carrier, and it is generally 50 µL to 300 µL, preferably 50 µL to 200 µL, and more preferably 50 µL to 150 µL per well in a case where the PS-affinitive substance is immobilized on a microplate.

The temperature at which the extracellular vesicle is brought into contact with the PS-affinitive substance is generally 2°C to 37°C and preferably 2°C to 30°C. The time of contact between the extracellular vesicle and the PS-affinitive substance is generally 0.5 to 24 hours, preferably 0.5 to 20 hours, and more preferably 0.5 to 12 hours.

As an example of the method of bringing the extracellular vesicle into contact with the PS-affinitive substance, a case where a Tim protein (a Tim carrier) immobilized on the insoluble carrier is used as the PS-affinitive substance will be described below.

In a case where a bead is used as the insoluble carrier of the Tim carrier, the followings are brought into contact with each other: a solution containing the extracellular vesicle obtained in the step (A-1), the amount thereof being generally 0.1 to 1,000 ml, preferably 0.1 to 500 ml, and more preferably 0.1 to 100 ml per 1 mg of the Tim carrier; the Tim carrier, the amount thereof being generally 0.001 to 20 mg, preferably 0.005 to 10 mg, and more preferably 0.01 to 6.0 mg per 1 mL of a solution after mixing, the solution containing the extracellular vesicle obtained in the step (A-1), the Tim carrier, and the solution containing calcium ions according to the embodiment of the present invention; and a solution containing calcium ions, the amount thereof being such that the concentration of calcium ions in a solution is generally 0.5 mM to 100 mM, preferably 1.0 mM to 10 mM, and more preferably 2.0 mM to 5.0 mM, where the solution is a solution after mixing the solution containing the extracellular vesicle, the Tim carrier, and the solution containing calcium ions according to the embodiment of the present invention. The PS extracellular vesicle and the Tim protein immobilized on the bead carrier are brought into contact with each other generally at 2°C to 37°C and preferably 2°C to 30°C, generally for 0.5 to 24 hours, preferably 1 to 20 hours, and more preferably 1 to 12 hours, thereby forming a complex thereof.

In a case where a microplate is used as the insoluble carrier of the Tim carrier, the solution containing calcium ions according to the embodiment of the present invention, the amount of which is such that the concentration of calcium ions in a solution is generally 0.5 mM to 100 mM, preferably 1.0 mM to 50 mM, and more preferably 2.0 mM to 50 mM, where the solution is the solution at the time of brining the Tim carrier into contact with the solution containing the extracellular vesicle obtained in the (A-1) step, and the solution containing the extracellular vesicle obtained in the step (A-1), the amount of which is generally 50 µL to 300 µL and preferably 100 µL to 200 µL per well, are added in each well of the microplate on which the Tim protein is immobilized, and the resultant mixture is reacted generally at 2°C to 37°C and preferably 2°C to 30°C, generally for 0.5 to 24 hours, preferably 0.5 to 20 hours, and more preferably 0.5 to 12 hours, thereby forming a complex of the PS extracellular vesicle and the Tim protein immobilized on the microplate carrier.

### (4-2) Complex 2 formation: reaction of complex of PS extracellular vesicles and PS-affinitive substance with anti-PSMA antibody

After obtaining a complex of the PS extracellular vesicle and the PS-affinitive substance, the obtained complex is brought into contact and reacted with the anti-PSMA antibody to obtain the complex 2.

The concentration of the anti-PSMA antibody that is used in the complex 2 formation step may be appropriately selected from the concentration range that is generally used in the immunological measurement in the related field.

The dilution rate of the anti-PSMA antibody that is reacted with the complex of the PS extracellular vesicle and the PS-affinitive substance varies depending on the activity and the concentration of the antibody; however, it is generally 10 to 1,000,000 times and preferably 1,000 to 100,000 times.

A diluent for the anti-PSMA antibody may be any one that does not interfere with the binding between the complex of the PS-affinitive substance and the PS extracellular vesicle, and the anti-PSMA antibody except that it contains calcium ions. Examples thereof include water and a buffer solution having a buffering action at a pH of 7.0 to 8.0 and preferably at a pH of 7.2 to 7.6 (for example, TBS, HBS, or the like). It is noted that the phosphate buffer is not preferable since it binds to calcium and causes precipitation. In addition, the buffering agent concentration in these buffer solutions is appropriately selected generally from a range of 5 to 50 mM and preferably 10 to 30 mM, and the concentration of NaCl is added is appropriately selected generally from a range of 100 to 200 mM and preferably from a range of 140 to 160 mM. This solution may contain, for example, saccharides, salts such as NaCl, a surfactant, a preservative, and a protein such as BSA, as long as the amounts thereof do not interfere with the binding between the complex of the PS-affinitive substance and the PS extracellular vesicle, and the anti-PSMA antibody. Examples of the surfactant include Tween20, and the concentration of the surfactant in the above solution is generally 0.00001% to 0.2% and preferably 0.0005% to 0.1%. The calcium ion-containing concentration of the diluent is generally 0.5 to 100 mM, preferably 1 to 10 mM, and more preferably 2 to 5 mM.

In a case where the PS-affinitive substance is immobilized on a bead, the amount of the anti-PSMA antibody solution that is reacted with the complex of the PS extracellular vesicle and the PS-affinitive substance is generally 0.1 mL to 1,000 mL, preferably 0.1 mL to 500 mL, and more preferably 0.1 mL to 100 mL with respect to 1 mg of the carrier, and it is generally 50 µL to 300 µL, preferably 50 µL to 200 µL, and more preferably 50 µL to 150 µL per well in a case where the PS-affinitive substance is immobilized on a microplate.

The temperature at which the complex of the PS extracellular vesicle and the PS-affinitive substances is brought into contact with the anti-PSMA antibody is generally 2°C to 37°C, preferably 10°C to 37°C, and more preferably 20°C to 30°C. Further, the time of reaction between the anti-PSMA antibody and the complex is generally 0.5 to 12 hours, preferably 1 to 4 hours, and more preferably 1 to 2 hours.

After the complex 2 formation step, the complex 2 which is "the complex of the PS-affinitive substance immobilized on the insoluble carrier, the PS-PSMA extracellular vesicle, and the labeled anti-PSMA antibody] obtained in the complex 2 formation step] may be subjected to, before the measurement step (A-3), a step of separating it from the reaction solution.

This complex 2 separation step may be any method as long as it can separate the complex 2 from the reaction solution, in other words, as long as the so-called B/F separation is possible, and a B/F separation method that is used in the related field can be used. Specifically, it may be the same method as the complex 1 separation step in the acquisition method of the step of (A-1-3).

### (5) Washing operation

A washing operation is optionally carried out appropriately between steps of the (A-2) according to the embodiment of the present invention. However, the washing operation is carried out in the presence of calcium ions. For example, a general washing operation may be carried out using the "calcium ion-containing washing solution" described in the above-described complex 1 separation step of (A-1-2).

### • (A-3) Measurement step

The measurement step (A-3) is "a step of measuring an amount of the complex 2 obtained in the step (A-2) to measure an amount of the PS-PSMA extracellular vesicle".

Examples of the method of measuring the amount of the complex 2 according to the embodiment of the present invention include enzyme immunoassay (EIA), radioimmunoassay (RIA), enzyme-linked immunosorbent assay (ELISA), fluoroimmunoassay (FIA), a measurement method by simple immunochromatography, high performance liquid chromatography (HPLC), an electrophoresis method, a capillary electrophoresis method, capillary chip electrophoresis method, a mass spectrometry method, an immunonephelometry method, and an immunoturbidimetry method. Among them, enzyme immunoassay (EIA) or enzyme-linked immunosorbent assay (ELISA) is preferable, and enzyme-linked immunosorbent assay (ELISA) is more preferable.

Examples of the principle of these measurements include a sandwich method, a competitive method, and a two-antibody method, and, and a sandwich method is preferable. Further, it is also possible to carry out measurement by a heterogeneous method in which the B/F separation is carried out using an insoluble carrier or the like, or it is also possible to carry out measurement by a homogeneous method in which the B/F separation is not carried out.

In a case of measuring the amount of "the complex 2 of the PS-affinitive substance, the PS-PSMA extracellular vesicle, and labeled anti-PSMA antibody" generated, by using the labeled anti-PSMA antibody in the step (A-2), it suffices that the labeling amount derived from the labeled anti-PSMA antibody in the complex 2 is measured. The method of measuring the labeling amount differs depending on the kind of the labeling substance, and thus it may be carried out according to a predetermined method according to the properties of the labeling substance.

For example, in a case where the labeling substance is an enzyme, the measurement may be carried out according to a conventional method of the immunoassay, for example, the method described in "Enzyme Immunoassay" (Protein, nucleic acid and enzyme, separate volume No. 31, edited by Tsunehiro Kitagawa, Toshio Minamihara, Akio Tsuji, Eiji Ishikawa, 51 to 63, Kyoritsu Shuppan Co., Ltd., 1987) or the like.

In a case where the labeling substance is a radioactive substance, the measurement may be carried out, for example, according to a common method that is carried out in RIA, by appropriately selecting and using a measurement instrument such as an immersion type GM counter, a liquid scintillation counter, a well type scintillation counter, or a counter for HPLC, depending on the kind and the intensity of radiation generated by the radioactive substance (for example, refer to "Medical Chemistry Experimental Course", Vol. 8, supervised by Yuichi Yamamura, First edition, Nakayama Shoten Co., Ltd., 1971).

In a case where the labeling substance is a fluorescent substance, the measurement may be carried out, for example, according to a conventional method that is used in FIA using a measurement instrument such as a fluorometer, for example, the method described in "Fluorescent Antibody with Illustrations, written by Akira Kawao, First edition, Soft Science Co., Ltd., 1983".

In a case where the labeling substance is a luminescent substance, the measurement may be carried out by using a measurement instrument such as a microplate reader such as En Vision (manufactured by PerkinElmer, Inc.), or a photo counter, and a measuring method in response to each measurement instrument.

In a case where the labeling substance is a substance having absorption in the ultraviolet region, the measurement may be carried out by a conventional method using a measurement instrument such as a spectrophotometer. In a case where the labeling substance has spin properties, the measurement may be carried out according to a conventional method using an electron spin resonance device, for example, the method described in "Enzyme Immunoassay" (Protein, nucleic acid and enzyme, separate volume No. 31, edited by Tsunehiro Kitagawa, Toshio Minamihara, Akio Tsuji, Eiji Ishikawa), 264 to 271, Kyoritsu Publishing Co., Ltd., 1987) or the like.

In a case where the labeling substance is an enzyme, examples of the measurement include a method known per se, such as a method of reacting the enzyme with a color developing reagent to induce a color developing reaction and then measuring the amount of a coloring agent generated as a result of the reaction with a spectrophotometer or the like. It is noted that in order to stop the color developing reaction, a reaction terminating method that is generally carried out in the related field may be used, where the reaction terminating method includes adding to the reaction solution, for example, an enzyme activity inhibitor such as 1 to 6 N sulfuric acid or a reaction terminating agent attached to the kit.

in a case of using alkaline phosphatase as the labeling substance, examples of the color developing reagent include dioxetane-based chemiluminescent substrates of alkaline phosphatase, such as CDP-Star^{™} (Tropix, Inc., product name, disodium 2-chloro-5-(4-methoxyspiro [1,2-dioxetane-3,2'-(5-chlorotricyclo[3.3.1.13.7]decane])-4-yl]-1-phenyl phosphate), AMPPD, and CSPD. In a case of using peroxidase as the labeling substance, examples thereof include a tetramethylbenzidine (TMB) solution and an orthophenylene diamine (OPD) solution, and a TMB solution is preferable. In addition, examples thereof include color developing reagents generally used in the related art, such as 3,3',5,5'-tetramethylbenzidine (TMBZ), o-phenylene diamine, o-nitrophenyl-β-D-galactoside, 2,2'-azino-bis (3-ethylbenzthiazoline-6-sulfonic acid) (ABTS), N-ethyl-N-sulfopropyl-m-anisidine (ADPS), and p-nitrophenyl phosphate.

All the reagent to be used in the method of measuring the amount of the PS-PSMA extracellular vesicles according to the embodiment of the present invention, the concentration thereof at the time of measurement, and the measurement conditions and the like (such as the reaction temperature, the reaction time, the pH at the time of reaction, the measurement wavelength, and measurement device) at the time of carrying out the measurement may be appropriately selected according to the measurement operation method of the same immunological measuring method as described above, which is a method known per se.

The measurement step of the step (A-3) is not limited to the manual method, and it may be carried out by a measurement system using an automatic analysis apparatus. It is noted that the are no particular restrictions on the combination of reagents or the like in a case of carrying out the measurement using a manual method or an automatic analysis apparatus, and a combination of reagents or the like which is considered to be the best combination may be selected and used appropriately, depending on the environment and the model of the automatic analysis apparatus to be applied, or in consideration of other factors.

As a specific example of the method of measuring the amount of the PS-PSMA extracellular vesicles according to the embodiment of the present invention, a method of measuring the amount of PS-PSMA extracellular vesicles in a specimen by using a PS-affinitive substance immobilized on an insoluble carrier and an anti-PSMA antibody labeled with alkaline phosphatase (ALP) is described below.

For example, in a case where it is carried out using a PS-affinitive substance immobilized on a microplate by the ELISA method, after forming a complex of the PS extracellular vesicle and the PS-affinitive substance immobilized on the microplate, and as necessary, a washing operation is carried out, a diluted solution obtained by diluting an ALP-labeled anti-PSMA antibody with a diluent for the anti-PSMA antibody containing calcium ions generally 0.5 to 100 mM, preferably 1 to 50 mM, and more preferably 2 to 50 mM, by generally 10 to 1,000,000 times and preferably 1,000 to 100,000 times is added in each well of the microplate, where the amount of the diluted solution is generally 50 µL to 300 µL, preferably 50 µL to 200 µL, and more preferably 50 µL to 100 µL per well, and the reaction is carried out generally at 2°C to 37°C and preferably 10°C to 37°C, for 0.5 to 12 hours and preferably 1 to 4 hours.

Next, each well is washed with a calcium ion-containing washing solution.

Further, an ALP enzyme reaction substrate solution such as the CDP-Star Substance with Emerald II Enhancer (manufactured by Thermo Fisher Scientific, Inc.) is added in each well, where the amount thereof is generally 50 µL to 300 µL, preferably 50 µL to 200 µL, and more preferably 50 µL to 100 µL per well, and the reaction is carried out at 2°C to 37°C and preferably 20°C to 30°C, for 5 minutes to 60 minutes and preferably 10 minutes to 40 minutes. Then, the chemiluminescence signal may be measured using a measurement instrument such as a microplate reader such as EnVision (manufactured by PerkinElmer, Inc.).

Using the obtained measured values, a determination step for metastatic PSMA may be subsequently carried out.

### <(B) Determination step according to present invention>

The determination step according to the embodiment of the present invention is a step of measuring the amount of PS-PSMA extracellular vesicles by the measurement step according to the embodiment of the present invention and determining whether or not a subject suffers from metastatic CRPC based on the obtained measurement results.

That is, the amount of PS-PSMA extracellular vesicles in the biological specimen derived from a subject is measured by the method described in the section of "(A) Measurement step according to present invention" described above, and based on the measurement result, the data on the PS-PSMA extracellular vesicle for determining metastatic CRPC (for example, the information such as the presence or absence, the concentration, the degree of increase in amount, or the like of the PS-PSMA extracellular vesicle) is obtained. Using the obtained data, the determination (the diagnosis or the examination) of metastatic CRPC is carried out, for example, by the following method.

For example, in a case where a reference value (a cutoff value) is set in advance and the measurement result (the measured value) of the PS-PSMA extracellular vesicle is equal to or larger than the reference value, it is possible to determine that, for example, the subject who has provided the specimen has a possibility of suffering from metastatic CRPC, has a high possibility of suffering from metastatic CRPC, has a possibility of transition to metastatic CRPC, or has a high probability of transition to metastatic CRPC.

It suffices that the amounts of PS-PSMA extracellular vesicles in specimens are measured according to the above-described measuring method, by using specimens derived from a metastatic CRPC patient and a patient having a prostate disease other than the metastatic CRPC (non-metastatic CRPC, prostate cancer other than CRPC, or benign prostatic hyperplasia) or healthy subject, and the reference value (the cutoff value) is set based on the boundary values of the measured values or the like. The average value of the amounts of PS-PSMA extracellular vesicles in patients having a prostate disease other than the metastatic CRPC disease (non-metastatic CRPC, prostate cancer other than CRPC, or benign prostatic hyperplasia) or healthy subjects may be set as the reference value.

Further, the above reference value (the cutoff value) can be determined based on the statistical analysis such as the receiver operating characteristic (ROC) curve analysis, by using a measured value obtained by the measurement step according to the embodiment of the present invention using a specimen derived from a subject suffering from metastatic CRPC, and by using a value (he, may be abbreviated as a healthy subject-derived value) obtained by the measurement step according to the embodiment of the present invention using a specimen derived from a patient having a prostate disease other than the metastatic CRPC (non-metastatic CRPC, prostate cancer other than CRPC, or benign prostatic hyperplasia) or a healthy subject.

In addition, the sensitivity or specificity of the reference value (the cutoff value or the like) is, for example, 60% or more, preferably 70% or more, more preferably 80% or more, and still more preferably 90% or more.

Another form includes a method of setting a plurality of determination categories in response to the amount of PS-PSMA extracellular vesicles in the specimen or the quantitative range thereof (the measured value or the range of the measured value) and carrying out determination. For example, determination categories [such as [(1) there is no risk of non-metastatic CRPC, (2) there is a low risk of non-metastatic CRPC, (3) there is a risk of non-metastatic CRPC, and (4) there is a high risk of non-metastatic CRPC] are set. Then, the determination of the non-metastatic CRPC can be made by determining which determination category the measurement result of the amount of PS-PSMA extracellular vesicles in the biological specimen derived from a subject belongs to.

As still another form, in the same subject, the measurement result of PS-PSMA extracellular vesicles in the subject-derived biological specimen measured at a certain time point is compared with the measurement result of PS-PSMA extracellular vesicles in the subject-derived biological specimen measured at a different time point, and the increase/decrease and/or degree of increase/decrease in the measurement result (the measured value) is evaluated, whereby the determination can be carried out. For example, in a case where an increase in the measurement result (the measured value) is observed, it is possible to determine that a pathological condition of a subject who has provided the specimen has progressed to the metastatic CRPC.

In a case of being determined that there is a possibility or high possibility that a patient who is the subject suffers from metastatic CRPC by a method of assisting the determination of metastatic CRPC of the present invention, it is possible to select further carrying out an invasive test such as a histopathological tissue test and various imaging examinations including the use of any one or a combination of a contrast agent, an isotope, a small molecule compound, an antibody, or the like. In addition, it is also possible to select the change of the treatment method.

On the other hand, in a case of being determined that there is no possibility or a low possibility that a patient who is the subject suffers from metastatic CRPC by the assisting method of the present invention, it is possible to select the treatment policy such as the continuation of the same treatment, the addition of topical treatment, or the suspension of treatment.

### <5. Examination kit for assisting diagnosis of metastatic CRPC according to embodiment of present invention>

An examination kit for assisting the diagnosis of metastatic CRPC of the present invention (hereinafter, may be abbreviated as a "kit of the present invention") is an examination kit containing a PS-affinitive substance and an anti-PSMA antibody as constitutional elements, which is used for measuring PS-PSMA extracellular vesicles. The PS-affinitive substance and the anti-PSMA antibody may be each in a solution state, a frozen state, a dry state, or a freeze-dried state.

The above-described "PS-affinitive substance" may be one bound to an insoluble carrier. The PS-affinitive substance and the PS-affinitive substance bound to an insoluble carrier are as described in the section of <3. Substance having affinity for phosphatidylserine>, and the same applies to specific examples thereof, the insoluble carrier, and the method of binding to the insoluble carrier, as well as preferred examples thereof.

Moreover, the "anti-PSMA antibody" may be a "labeled anti-PSA antibody". The anti-PSMA antibody and the labeled anti-PSMA antibody are described in the section of "(A-2) and (2) Antibody having affinity for PSMA" in <4. Method of assisting diagnosis of metastatic CRPC of present invention>, and the same applies to specific examples thereof, the labeling substance, and the labeling methods, as well as preferred examples thereof.

The kit of the present invention may further contain, as a constitutional element, an insoluble carrier on which the PS-affinitive substance that is used for acquiring PS extracellular vesicles is immobilized. The PS-affinitive substance and the PS-affinitive substance bound to an insoluble carrier are as described in the section of <3. Substance having affinity for phosphatidylserine>, and the same applies to specific examples thereof, the insoluble carrier, and the method of binding to the insoluble carrier, as well as preferred examples thereof.

Further, the kit of the present invention may contain at least one selected from the calcium ion-containing solution, the calcium ion-containing washing solution, or the calcium ion chelating agent-containing solution. Specific examples, the preferred form, or the like of each constitutional element are as described above.

Specific examples of the kit of the present invention include a kit (which shall be referred to as a kit A) containing any one of the following constitutional elements that are used for measuring PS-PSMA extracellular vesicles.
- A solid phase plate on which the PS-affinitive substance is immobilized and the anti-PSMA antibody labeled with a labeling substance
- A solid phase plate on which the PS-affinitive substance labeled with a labeling substance and the anti-PSMA antibody are immobilized.

Other specific examples of the kit of the present invention include a kit (which shall be referred to as a kit B) containing one selected from the following constitutional elements that are used for acquiring PS-PSMA extracellular vesicles.
- A bead carrier on which the PS-affinitive substance is immobilized (a reagent that is used to acquire PS extracellular vesicles from a biological specimen)
- A solution containing calcium ions
- A calcium ion-containing washing solution
- A calcium ion chelating agent

Still other specific examples of the kit of the present invention include the kit A and the kit B.

Preferred specific examples of the kit of the present invention include a kit including the following constitutional elements of (1) or (2).
(1) A solid phase plate on which the PS-affinitive substance is immobilized and the anti-PSMA antibody labeled with a labeling substance, or a solid phase plate on which the PS-affinitive substance labeled with a labeling substance and the anti-PSMA antibody are immobilized.
(2) • A solid phase plate on which the PS-affinitive substance is immobilized and the anti-PSMA antibody labeled with a labeling substance, or a solid phase plate on which the PS-affinitive substance labeled with a labeling substance and the anti-PSMA antibody are immobilized, and
   - a bead carrier on which the PS-affinitive substance is immobilized, which is used for acquiring PS-PSMA extracellular vesicles.

The constitutional elements of each of the above kits are as described above, and the same applies to specific examples and preferred examples thereof.

In addition, the reagent included in these kits may have those that are generally used in the related field, for example, a buffering agent, a sensitizer, a surfactant, a preservative (for example, sodium azide, salicylic acid, or benzoic acid), a stabilizer (for example, albumin, globulin, water-soluble gelatin, surfactant, or saccharides), an activator agent, an agent for avoiding the influence of coexisting substances, and a reagent for detecting a labeling substance, where these reagents do not inhibit the stability of coexisting reagents, the reaction or binding between the PS-affinitive substance and the extracellular vesicles, and the reaction or binding between the anti-PSMA antibody and the extracellular vesicle. Further, regarding the concentration range, pH, and the like of these reagents or the like, the concentration range, pH, and the like, which are generally used in order to exhibit the effect of each of the reagents, may be appropriately selected and used.

In a case where the anti-PSMA antibody is labeled with a labeling substance, the above reagent for detecting a labeling substance is a reagent that detects the label, and it is appropriately selected depending on the kind of the labeling substance. Examples thereof include a substrate for measuring absorbance such as tetramethylbenzidine or orthophenylene diamine, a fluorescent substrate such as hydroxyphenyl propionic acid or hydroxyphenyl acetic acid, a luminescent substance such as CDP-Star TM or luminol, a reagent for measuring absorbance such as 4-nitrophenyl phosphate, and a fluorescent substrate such as 4-methylumbelliferyl phosphate.

Furthermore, the kit of the present invention may include an instruction manual or the like for carrying out the assisting method of the present invention. The "instruction manual" means a user's manual, attached document, pamphlet (leaflet), or the like for the reagents contained in the kit according to the embodiment of the present invention in which the feature, the principle, the operating procedure, the determination procedure, and the like of the assisting method of the present invention are substantially described in sentences and/or illustrated. Specifically, examples thereof include (i) an instruction manual in which the principle of the measurement step according to the embodiment of the present invention and the operating procedure thereof are described, and (ii) an instruction manual in which the principle, the operating procedure, and the like of the measurement step and the determination step according to the embodiment of the present invention are described.

In a case where the kit of the present invention is used, the assisting method of the present invention can be carried out easily, in a short time, and with high accuracy.

### <6. Biomarker according to embodiment of present invention for assisting diagnosis of metastatic CRPC according to embodiment of present invention>

The biomarker of the present invention for assisting the diagnosis of metastatic CRPC is the extracellular vesicle (the PS-PSMA extracellular vesicle) having phosphatidylserine and PSMA on the membrane surface thereof. The details of the extracellular vesicle are as described in the section of "<1. Extracellular vesicles according to present invention>".

### <7. Device for assisting diagnosis of metastatic CRPC according to present invention>

The device for assisting diagnosis of metastatic CRPC according to the embodiment of the present invention (hereinafter, abbreviated as an "assisting device according to the embodiment of the present invention") includes at least (1) a measurement unit. It may further include (2) a determination unit, (3) an output unit, and (4) an input unit.

The measurement unit in the assisting device according to the embodiment of the present invention is configured to measure the amount of PS-PSMA extracellular vesicles in a biological specimen derived from a subject, where the PS-PSMA extracellular vesicle is the biomarker of the present invention. Specific examples of the assisting device include a measurement device such as a device that is used in a method according to the immunological measuring method.

It is noted that, as necessary, the measurement unit may be configured to calculate the amount of the biomarker of the present invention based on the measured value.

The determination unit in the assisting device according to the embodiment of the present invention is configured to determine whether or not the measured value obtained by the measurement in the measurement unit is equal to or larger than the reference value.

Alternatively, the determination unit in the assisting device according to the embodiment of the present invention is configured to determine whether a subject suffers from metastatic CRPC using, as indicators, the measurement unit that measures the amount of PS-PSMA extracellular vesicles in a biological specimen derived from a subject obtained by the measurement unit, and the amount of the PS-PSMA extracellular vesicles.

For example, it may be configured such that the measured value measured by the measurement unit is compared with the preset reference value (cutoff value) to determine whether it is equal to or larger than the reference value or smaller than the reference value.

The output unit in the assisting device according to the embodiment of the present invention is configured to output the result obtained by the measurement unit or/and the result obtained by the determination unit.

The input unit in the assisting device according to the embodiment of the present invention is configured to send a signal for operating the measurement unit to the measurement unit in response to an operation by an operator.

The device of the above (1) to (4), which constitute the assisting device according to the embodiment of the present invention, may be arranged in the same device or may be each a separate body.

In the assisting device according to the embodiment of the present invention, the subject, the biological specimen, and the PS-PSMA extracellular vesicles, which are related to the assisting device, are as described above, and the same applies to specific examples and preferred examples thereof.

The measurement, the determination, and the like carried out by the measurement unit and the determination unit of the assisting device according to the embodiment of the present invention are as described in <4. Method of assisting diagnosis of metastatic CRPC according to embodiment of present invention>, and the same applies to preferred examples, specific examples, and the like thereof.

In a case where the assisting device according to the embodiment of the present invention is used, the assisting method of the present invention can be carried out easily, in a short time, and with high accuracy.

### <8. Method of treating metastatic CRPC according to present invention>

A method of treating metastatic CRPC according to the embodiment of the present invention (hereinafter, abbreviated as a "treatment method according to the embodiment of the present invention") is carried out by measuring the amount of the biomarker of the present invention in a biological specimen derived from a subject, determining whether or not the subject suffers from metastatic CRPC based on the measurement result, and subjecting a patient who is at risk of metastatic CRPC or at high risk of metastatic CRPC to a suitable treatment based on the determination result.

The biological specimen derived from a subject, the marker, the measurement, the determination, and the like in the treatment method according to the embodiment of the present invention are as described in the section of <4. Method of assisting diagnosis of metastatic CRPC according to embodiment of present invention> described above, and the same applies to preferred examples, specific examples, and the like thereof.

Specific examples of the suitable therapy in the treatment method according to the embodiment of the present invention include a surgical treatment, a radiation therapy (including external irradiation, internal irradiation, and internal administration of an isotope), an anticancer chemical therapy (docetaxel, cabazitaxel, or the like), an androgen ablation therapy (leuprorelin, goserelin, degarelix, or the like), an anti-androgen therapy (an androgen receptor inhibitor: bicalutamide, flutamide, apalutamide, enzalutamide, or the like, an androgen production inhibitor: abiraterone, or female hormone: estramustine, estradiol, or the like), and a treatment method with another drug (an immune checkpoint inhibitor: pembrolizumab or the like, a molecular target drug, or the like).

Hereinafter, the present invention will be described in more detail with reference to Examples and Comparative Examples; however, the present invention is not limited to these Examples and the like.

### Examples

### Example 1. Measurement of PSMA extracellular vesicle

### (1) Acquisition of Tim4-immobilized bead

A TBS-T solution (Tris buffer, 0.01% Tween 20) containing 125 ng per specimen of the Tim4 protein (manufactured by FUJIFILM Wako Pure Chemical Corporation) was added in a 1.5 mL tube in which 75 µg per specimen of Dynabeads MyOne Streptavidin C1 (manufactured by Thermo Fisher Scientific, Inc.) were aliquoted, the reaction was carried out at room temperature for 1 hour, and then washing was carried out 5 times with a TBS-T solution containing 0.1% BSA to obtain beads (Tim4 beads) to which the Tim4 protein had been bound.

### (2) Preparation of specimen

Using the Tim4 beads, a specimen containing extracellular vesicles (PS extracellular vesicles) having phosphatidylserine on the membrane surface was prepared by the following method.

50 µL per specimen of the TBS (the Tris buffer) containing 2% BSA to which CaCh had been added so that the final concentration was 4 mM was added to the Tim4 beads obtained in the above (1) and suspended. The obtained Tim4 bead suspension was aliquoted into a 96-well polypropylene plate so that the amount thereof was 50 µL per well (75 µg of Tim4 beads per well, 125 ng in terms of Tim4).

50 µL of the serum derived from each of CRPC patients in which metastasis was confirmed (metastatic CRPC patients, n = 12), CRPC patients in which metastasis was not observed (non-metastatic CRPC patients, n = 2), prostate cancer patients confirmed not to be CRPC (n = 70), benign prostatic hyperplasia patients (n = 24), or healthy subjects (n = 10) was added in 96-well plate well in which the Tim4 bead suspension had been aliquoted, and the reaction was carried out for 1 hour with stirring at room temperature.

After the reaction, the 96-well plate was installed on a magnet stand for the 96-well plate, the Tim4 beads were collected on the tube wall using magnetic force, and the reaction solution was removed. Then, each well was subjected to the washing operation 5 times with 200 µL of the TBS-T containing 2 mM CaCl₂.

50 µL of the TBS containing 2 mM EDTA was added as an eluent to the washed Tim4 beads, and then stirring was carried out at room temperature for 10 minutes. Then, the 96-well plate was installed on a magnet stand for the 96-well plate, the Tim4 beads were collected on the tube wall using magnetic force, and the supernatant (the eluent) was collected.

The obtained supernatant was used as a specimen. This specimen contains extracellular vesicles (PS extracellular vesicles) having phosphatidylserine on the membrane surface thereof.

### (3) Measurement of extracellular vesicle by ELISA

The amount of the extracellular vesicle (the PS-PSMA extracellular vesicle) having phosphatidylserine and PSMA in the specimen was measured according to the methods 1) to 3) below.

### 1) Acquisition of Tim4 plate

100 µL per well of 1.25 µg/mL of Tim4 (a 50 mM carbonate buffer, pH 9.6) was aliquoted to a white MaxiSorp 96-well plate (Thermo Fisher Scientific, Inc.). After allowing to stand overnight at 4°C, the plate was washed 3 times with 400 µL of TBS-T (Trs buffer).

### 2) Acquisition of alkaline phosphatase (ALP)-labeled anti-PSMA antibody

The PSMA antibody (manufactured by Miltenyi Biotec) was subjected to ALP labeling by using an ALP labeling kit-SH (manufactured by Dojindo Molecular Technologies. Inc.) according to the user's manual attached to the kit.

### 3) ELISA

200 µL per well of the TBS containing 1% BSA was added to the Tim4 plate obtained in the above 1), and blocking was carried out with stirring at room temperature for 1 hour. Then, the wells were washed 3 times with 400 µL of TBS-T, and then 50 µL per well of TBS containing 2% BSA, to which CaCh was added so that the final concentration was 40 mM, was aliquoted thereto. The specimen prepared in the above (2) was added thereto, and the reaction was carried out with stirring at room temperature for 1 hour. After the reaction, each well was washed 5 times with 400 µL of the TBS-T containing 2 mM CaCl₂.

After washing, 100 µL per well of the ALP-labeled anti-PSMA antibody obtained in the above 2) was added to 0.25 µg/mL (TBS containing 1% BSA, containing 2 mM CaCh), the reaction was carried out with stirring at room temperature for 1 hour. Then, each well was washed 5 times with 400 µL of the TBS-T containing 2 mM CaCl₂.

After washing, 50 µL per well of CDP-Star Substance with Emerald-II Enhancer (manufactured by Thermo Fisher Scientific, Inc.) was added thereto, the reaction was carried out with stirring for 30 minutes, and then the chemiluminescence signal was measured using EnVision (manufactured by PerkinElmer, Inc.).

### 4) Result

The results are shown in Fig. 1. In Fig. 1, five specimens from CRPC patients of which the measured values are in the vicinity of the average value of the measured values of the metastatic CRPC patients are selected, the average value thereof is set to 1, and the relative value of the measured value of the specimen derived from each disease patient or healthy subject with respect to this average value is shown.

As revealed in Fig. 1, it was found that the value of the amount of PS-PSMA extracellular vesicles in the metastatic CRPC patients (with CRPC metastasis) is significantly high as compared with those of all the non-metastatic CRPC patients (without CRPC metastasis), the prostate cancer patients (PC) confirmed to be non-metastatic CRPC, the benign prostatic hyperplasia (BPH) patients, and the healthy subjects (Normal).

From the above results, it was shown that the amount of PS-PSMA extracellular vesicles serves as a marker for determining metastatic CRPC and that it is possible to determine metastatic CRPC by measuring the amount of PS-PSMA extracellular vesicles.

### Comparative Example 1: Comparison with technique in related art

Among the same specimens used in Example 1, the serum of the benign prostatic hyperplasia patients (n = 8), the prostate cancer patients confirmed not to be CRPC (n = 8), the CRPC patients (the metastatic RPC patients and the non-metastatic CRPC patients) (n = 8), and the healthy subjects (n = 10) were used as specimens, specimens containing extracellular vesicles (PS extracellular vesicles) having phosphatidylserine on the membrane surface were prepared by the same method as in "(2) Preparation of specimen", and the concentration of the amount of PS-PSMA extracellular vesicles in the specimens was measured by the following method.

### (1) Preparation of reagents

### 1) Acquisition of anti-PSMA antibody-immobilized plate

100 µL per well of 1.25 µg/mL anti-PSMA antibody (Miltenyi Biotec) diluted in a 50 mM carbonate buffer pH 9.6 was aliquoted in a white MaxiSorp 96-well plate (manufactured by Thermo Fisher Scientific, Inc.). After allowing to stand overnight at 4°C, the plate was washed 3 times with 400 µL of TBS-T.

### 2) Acquisition of anti-CD9 antibody-immobilized plate

100 µL per well of 1.25 µg/mL anti-CD9 antibody (manufactured by BioLegend, Inc.) diluted in a 50 mM carbonate buffer pH 9.6 was aliquoted in a white MaxiSorp 96-well plate (manufactured by Thermo Fisher Scientific, Inc.). After allowing to stand overnight at 4°C, the plate was washed 3 times with 400 µL of TBS-T.

### 3) Acquisition of ALP-labeled anti-CD9 antibody

The CD9 antibody (manufactured by BioLegend, Inc.) was subjected to ALP labeling by using an ALP labeling kit-SH (manufactured by Dojindo Molecular Technologies. Inc.) according to the user's manual.

### 4) Acquisition of ALP-labeled anti-PSMA antibody

The PSMA antibody (manufactured by Miltenyi Biotec) was subjected to ALP labeling by using an ALP labeling kit-SH (manufactured by Dojindo Molecular Technologies. Inc.) according to the user's manual.

### (2) Measurement of PS-PSMA extracellular vesicle

### 1) Measurement system with immobilized anti-PSMA antibody-labeled anti-CD9 antibody

200 µL per well of the TBS containing 1% BSA was added in the anti-PSMA antibody-immobilized plate obtained in the above (1) 1), and blocking was carried out with stirring at room temperature for 1 hour. After washing the plate 3 times with TBS-T, TBS containing 2% BSA and specimens (50 µL each) were added thereto, and the reaction was carried out with stirring at room temperature for 1 hour. Then, the plate was washed 5 times with TBS-T, and 100 µL per well of the TBS containing 1% BSA, in which the ALP-labeled anti-CD9 antibody of the above (1) 3) was diluted to 0.25 µg/mL, was added thereto, and the reaction was carried out with stirring at room temperature for 1 hour. After the reaction, the plate was washed 5 times with TBS-T, 50 µL per well of CDP-Star Substance with Emerald-II Enhancer (manufactured by Thermo Fisher Scientific, Inc.) was added thereto, the reaction was carried out with stirring for 30 minutes, and then chemiluminescence signal was measured using EnVision (manufactured by PerkinElmer, Inc.).

### 2) Measurement system with immobilized anti-CD9 antibody-labeled anti-PSMA antibody

200 µL per well of the TBS containing 1% BSA was added in the anti-CD9 antibody-immobilized plate obtained in the above (1) 2), and blocking was carried out with stirring at room temperature for 1 hour. After washing the plate 3 times with TBS-T, TBS containing 2% BSA and specimens (50 µL each) were added thereto, and the reaction was carried out with stirring at room temperature for 1 hour. Then, the plate was washed 5 times with TBS-T, and 100 µL per well of the TBS containing 1% BSA, which contained 0.25 µg/mL of the ALP-labeled anti-PSMA antibody obtained in the above (1) 4), was added thereto, and the reaction was carried out with stirring at room temperature for 1 hour. After the reaction, the plate was washed 5 times with TBS-T. Next, 50 µL per well of CDP-Star Substance with Emerald-II Enhancer (manufactured by Thermo Fisher Scientific, Inc.) was added as an ALP enzyme reaction substrate solution, the reaction was carried out with stirring for 30 minutes, and then chemiluminescence was measured using EnVision (manufactured by PerkinElmer, Inc.).

### (3) Result

The results are shown in Fig. 2. In Fig. 2, the vertical axis indicates the luminescence intensity (cps).

(1) of Fig. 2 is the results of "Measurement system with immobilized anti-PSMA antibody-labeled anti-CD9 antibody" of the above (2) 1). In addition, (2) of Fig. 2 is the results of "Measurement system with immobilized anti-CD9 antibody-labeled anti-PSMA antibody" of the above (2) 2).

The method of the above (2) 1) is a method in which the method of detecting exosomes in Figure 3 of Kosuke Mizutani et al., Anticancer Research, 2014, 34(7), p.3419-3423 is converted to ELISA. In addition, the method of the above (2) 2) is a method in which the method of detecting exosomes in Figure 3 of Kosuke Mizutani et al., Anticancer Research, 2014, 34(7), p.3419-3423 is converted to ELISA, and the antibody immobilized on the solid phase and the labeled antibody are reversed.

As revealed in Fig. 2, both in a case of "Measurement system with immobilized anti-PSMA antibody-labeled anti-CD9 antibody" and a case of "Measurement system with immobilized anti-CD9 antibody-labeled anti-PSMA antibody"), there was no significant difference in the measured values between CRPC patients (CRPC), prostate cancer patients (PC) confirmed not to be CRPC, and benign prostatic hyperplasia (BPH) patients.

From the above results, it can be seen that CRPC patients cannot be distinguished from prostate cancer patients and benign prostatic hyperplasia patients even in a case of converting the method in the related art to ELISA and measuring exosomes (extracellular vesicles).

### Example 2

### (1) Preparation of specimen containing extracellular vesicle

After culturing C4-2B cells, which are a PSMA-positive human prostate cancer cell line, the culture supernatant was collected. 90 mL of the collected culture supernatant was further subjected to centrifugation treatment (1st time: 2,000 × g, 10 minutes, 2nd time: 12,000 × g, 30 minutes) to separate impurities, thereby obtaining a supernatant. The obtained culture supernatant sample was filtered through a filter (pore size: 0.22 µm) to separate impurities, thereby obtaining a supernatant.

Next, the obtained supernatant was subjected to ultracentrifugation treatment (100,000 × g, 70 minutes, 4°C) to obtain a precipitate fraction. The obtained precipitate fraction was suspended in PBS (-) and subjected to ultracentrifugation treatment (100,000 × g, 70 minutes, 4°C) again to wash the precipitate. After carrying out this washing operation again, the obtained precipitate fraction was suspended in 100 µL of PBS (-). The amount of protein in the specimen was measured by a conventional method.

### (2) Preparation of measurement specimen 1

A specimen containing the extracellular vesicles obtained in the above (1) was added to human pooled serum diluted to 50% with TBS so that the final concentration of protein amount at the time of measuring signal intensity was individually 0 ng/well, 0.39 ng/well, 0.78 ng/well, 1.56 ng/well, 3.12 ng/well, 6.25 ng/well, 12.5 ng/well, and 25 ng/well, and used as a measurement specimen 1. That is, the measurement specimen 1 is a human serum specimen containing extracellular vesicles.

### (3) Preparation of measurement specimen 2

A specimen containing the extracellular vesicles obtained in the above (1) was added to human pooled serum diluted to 50% with TBS so that the final concentration of protein amount at the time of measuring signal intensity was individually 0 ng/well, 0.39 ng/well, 0.78 ng/well, 1.56 ng/well, 3.12 ng/well, 6.25 ng/well, 12.5 ng/well, and 25 ng/well. Next, the same method as in "(2) Preparation of specimen" of Example 1 was carried out except that the human pooled serum to which the extracellular vesicles were added was used instead of the serum derived from the CRPC patient, and a supernatant containing extracellular vesicles having phosphatidylserine on the membrane surface thereof was obtained and used as a measurement specimen 2.

That is, the measurement specimen 2 is a specimen containing the PS extracellular vesicles obtained by separating PS extracellular vesicles from human serum containing extracellular vesicles using the Tim4 carrier (the Tim4 beads).

### (4) Measurement of extracellular vesicles by ELISA

The measurement specimen 1 obtained in the above (2) and the measurement specimen 2 obtained in the above (3) was used, and the amount (the signal intensity) of the PS-PSMA extracellular vesicles in each of the specimens was measured by the same method as in "(3) Measurement of extracellular vesicle by ELISA" of Example 1.

### (5) Result

The obtained results are shown in Fig. 3.

In Fig. 3, - □ - indicates the result of measurement using the measurement specimen 1 obtained in the above (2). In addition, - × - indicates the result of measurement using the measurement specimen 2 obtained in the above (3).

As revealed in Fig. 3, it can be seen that in a case where PS extracellular vesicles in the serum are acquired using Tim4, which is the PS-affinitive substance according to the embodiment of the present invention, and subsequently the PS-PSMA extracellular vesicles according to the embodiment of the present invention are measured, it is possible to eliminate most of the background of measurement, derived from impurities and the like contained in the serum.

According to the present invention, it is possible to diagnose non-invasive metastatic CRPC.

In addition, in a case where PS-PSMA extracellular vesicles are measured according to the embodiment of the present invention in addition to the measurement of PSA in blood, and these values are monitored during hormone therapy treatment, this measurement and monitoring can be used as an assistance tool of diagnosis for the progress of CRPC including metastasis, and it is possible to reduce the oversight of metastatic CRPC diagnosis due to carrying out diagnosis only by the PSA measurement in the related art.

Moreover, according to the embodiment of the present invention, it is possible to avoid frequent imaging examinations that are carried out on patients who do not require imaging examinations, and as a result, unnecessary radiation exposure can be reduced although monitoring for diagnosing the progress of CRPC without an increase in PSA requires frequent imaging examinations.

In Japan, a clinical test of PSMA-PET using a compound having a high affinity for PSMA by radiolabeling started in September 2019. In a case where the amount of PS-PSMA extracellular vesicles according to the embodiment of the present invention is measured as a companion diagnostic before receiving PSMA-PET, and PSMA-PET is carried out in a case where the value of the amount is high, it is possible to contribute to the suppression of health care cost and the reduction of radiation exposure of patients.

Furthermore, it is expected to be used for the "PSMA-PET companion diagnostic" using the present invention.

## Claims

1. A method of assisting diagnosis of metastatic castration resistant prostate cancer, comprising:
measuring an amount of an extracellular vesicle having phosphatidylserine and a prostate specific membrane antigen in a biological specimen derived from a subject; and
determining whether or not the subject has metastatic castration resistant prostate cancer by using, as an indicator, the amount of the extracellular vesicle having phosphatidylserine and a prostate specific membrane antigen.

2. The method according to claim 1,
wherein the determination is determining that the subject has metastatic castration resistant prostate cancer in a case where the amount of the extracellular vesicle having phosphatidylserine and a prostate specific membrane antigen is equal to or larger than a reference value.

3. The method according to claim 1,
wherein the measurement is a measurement in which a substance having an affinity for phosphatidylserine and an antibody having an affinity for a prostate specific membrane antigen are used.

4. The method according to claim 1,
wherein the measurement includes the following steps (1) to (3);
(1) a step of obtaining an extracellular vesicle from a biological specimen derived from a subject,
(2) a step of bringing the extracellular vesicle obtained in the step (1) into contact with a substance having an affinity for phosphatidylserine and an antibody having an affinity for a prostate specific membrane antigen, to form a complex 2 of the substance having an affinity for phosphatidylserine, the extracellular vesicle having phosphatidylserine and a prostate specific membrane antigen, and the antibody having an affinity for a prostate specific membrane antigen, and
(3) a step of measuring an amount of the complex 2 obtained in the step (2) to measure an amount of the extracellular vesicle having phosphatidylserine and a prostate specific membrane antigen.

5. The method according to claim 1,
wherein the measurement includes the following steps (1) to (3);
(1) a step of bringing a biological specimen derived from a subject into contact with a substance having an affinity for phosphatidylserine to form a complex 1 of an extracellular vesicle having phosphatidylserine in the biological specimen and the substance having an affinity for phosphatidylserine, subsequently separating the extracellular vesicle having phosphatidylserine from the complex 1, and acquiring the extracellular vesicle having phosphatidylserine,
(2) a step of bringing the extracellular vesicle having phosphatidylserine, obtained in the step (1), into contact with a substance having an affinity for phosphatidylserine and an antibody having an affinity for a prostate specific membrane antigen, to form a complex 2 of the substance having an affinity for phosphatidylserine, the extracellular vesicle having phosphatidylserine and a prostate specific membrane antigen, and the antibody having an affinity for a prostate specific membrane antigen, and
(3) a step of measuring an amount of the complex 2 obtained in the step (2) to measure an amount of the extracellular vesicle having phosphatidylserine and a prostate specific membrane antigen.

6. The method according to claim 5,
wherein in the step (1), the substance having an affinity for phosphatidylserine is bound to an insoluble carrier.

7. The method according to claim 5,
wherein the step (1) includes the following steps;
(1-1) a step of bringing the biological specimen into contact with a substance having an affinity for phosphatidylserine to form a complex 1 of an extracellular vesicle having phosphatidylserine in the biological specimen and the substance having an affinity for phosphatidylserine,
(1-2) a step of separating and acquiring the complex 1 obtained in the step (1-1) from the biological specimen, and
(1-3) a step of separating the extracellular vesicle having phosphatidylserine from the complex 1 obtained in the step (1-2) and acquiring the extracellular vesicle having phosphatidylserine.

8. The method according to claim 3,
wherein the substance having an affinity for phosphatidylserine is a T-cell immunoglobulin-mucin-domain containing protein.

9. The method according to claim 8,
wherein the T-cell immunoglobulin-mucin-domain containing protein is a T-cell immunoglobulin-mucin-domain containing protein 1 or a T-cell immunoglobulin-mucin-domain containing protein 4.

10. The method according to claim 1,
wherein the biological specimen is a blood specimen.

11. An examination kit for assisting diagnosis of metastatic castration resistant prostate cancer, the kit comprising a substance having an affinity for phosphatidylserine and an antibody having an affinity for a prostate specific membrane antigen, which are used for measuring an extracellular vesicle having phosphatidylserine and a prostate specific membrane antigen.

12. The kit according to claim 11, further comprising an insoluble carrier on which a substance having an affinity for phosphatidylserine is immobilized, where the substance is used for acquiring an extracellular vesicle having phosphatidylserine.

13. A diagnostic biomarker for metastatic castration resistant prostate cancer, the diagnostic biomarker comprising an extracellular vesicle having phosphatidylserine and a prostate specific membrane antigen.

14. A device for assisting diagnosis of metastatic castration resistant prostate cancer, the device comprising a measurement unit that measures an amount of an extracellular vesicle having phosphatidylserine and a prostate specific membrane antigen in a biological specimen derived from a subject.

15. The device according to claim 14, further comprising a determination unit that determines whether or not a measured value obtained by the measurement in the measurement unit is equal to or larger than a reference value.
